# EUROPÄISCHE PATENTSCHRIFT

(11) **EP 2 451 815 B1**
(45) Veröffentlichungstag und Bekanntmachung des Hinweises auf die Patenterteilung: **25.09.2013**
(21) Anmeldenummer: 10740516.9
(22) Anmeldetag: 09.07.2010
(51) Int. Cl.: C07D 487/18, A61K 31/407, A61P 25/28, A61P 31/00, A61P 35/00

(54) **INDUKTION VON alpha-HELIX-KONFORMATIONEN IN PROTEINEN UND PEPTIDEN**
INDUCTION OF alpha-HELIX CONFORMATIONS IN PROTEINS AND PEPTIDES
INDUCTION DE CONFORMATIONS EN HÉLICE alpha DANS DES PROTÉINES ET PEPTIDES

(30) Priorität: 10.07.2009 DE 102009032902
(43) Veröffentlichungstag der Anmeldung: 16.05.2012
(73) Patentinhaber: Universität zu Köln, 50923 Köln (DE); Forschungsverbund Berlin E.V., 12439 Berlin (DE)
(72) Erfinder: SCHMALZ, Hans-Günther, 50321 Brühl (DE); KUHNE, Ronald, 12683 Berlin (DE); HACK, Verena, 50937 Köln (DE); REUTER, Cédric, 50996 Köln (DE)
(74) Vertreter: Bülle, Jan
(86) Internationale Anmeldenummer: PCT/EP2010/004213
(87) Internationale Veröffentlichungsnummer: WO 2011/003626

(56) Entgegenhaltungen:
- WO-A1-2008/040332
- KEMP D S ET AL: "Studies of N-terminal templates for .alpha.-helix formation. Synthesis and conformational analysis of (2S,5S,8S,11S)-1-acetyl-1,4-diaza-3-keto-5 -carboxy-10-thiatricyclo[2.8.1.04,8]tridec ane (Ac-Hel1-OH)" JOURNAL OF ORGANIC CHEMISTRY, Bd. 56, Nr. 23, 1991, Seiten 6672-6682, XP002603314 ISSN: 0022-3263
- ZAMINER JAN ET AL: "Addressing protein-protein interactions with small molecules: a Pro-Pro dipeptide mimic with a PPII helix conformation as a module for the synthesis of PRD-binding ligands." ANGEWANDTE CHEMIE (INTERNATIONAL ED. IN ENGLISH), Bd. 49, Nr. 39, 17. September 2010 (2010-09-17), Seiten 7111-7115, XP002603315 ISSN: 1521-3773

## Beschreibung

Die vorliegende Erfindung betrifft substituierte tricyclische Diprolin-Derivate, Verfahren zu ihrer Herstellung, ihre Verwendung zur Induktion einer α-Helix-Konformation in Peptiden und/oder Proteinen, Arzneimittel enthaltend diese Verbindungen, die Verwendung dieser Verbindungen zur Herstellung von Arzneimitteln, Verfahren zur Herstellung von Peptid-Bibliotheken enthaltend diese Verbindungen und Peptid-Bibliotheken enthaltend diese Verbindungen.

Peptide und Proteine sind essentielle Bestandteile von Organismen mit einer Vielzahl verschiedener Funktionen. Während den Proteinen vor allem biokatalytische Aufgaben (Enzyme) sowie solche als 10 wichtige Gewebebestandteile zukommen, erfüllen die Peptide vor allem als Hormone, Neurotransmitter und Neuromodulatoren wichtige Funktionen im Organismus. Durch Bindung an Rezeptoren (bzw. an Proteinober-flächen oder Proteinkomplexe) beeinflussen Peptide nicht nur Enzymfunktionen, sondern auch spezifische makromolekulare Interaktionen (z.B. die Zell-Zell-Kommunikation oder Protein-Protein-Wechselwirkungen) und dadurch vermittelte zellphysiologische Folgereaktionen. So kontrollieren Peptide eine Vielzahl vitaler Prozesse wie Stoffwechsel, Immunabwehr, Verdauung, Atmung, Schmerzempfinden, Reproduktion, Verhalten, Elektrolythaushalt und mehr.

Die Wechselwirkung von Peptidliganden mit Proteinrezeptoren spielt eine wichtige Rolle bei der Regulation biologischer Prozesse und hängt in entscheidender Weise von der Peptidgeometrie ab. Unter physiologischen Bedingungen steht die Konfor-mation eines linearen Peptids durch Rotation um einzelne Bindungen in einem dynamischen Gleichgewicht, das vom pH-Wert und von der Temperatur abhängt. Dies führt dazu, dass die biologische Reaktivkonformation nur zu einem geringen Prozentsatz vorliegt.

Die Konformation des Peptidrückgrats wird üblicherweise durch die drei Winkel Φ, Ψ und w beschrieben. Aufgrund des partiellen Doppelbindungscharakters ist die Peptidbindung in ihrer Rotation gehindert und weist eine planare Geometrie auf, was zu zwei Vorzugskonformationen führt: der *trans* und der cis-Peptidbindung mit w = 180° bzw. ω = 0°, wobei die trans-Konformation energetisch günstiger ist und deshalb überwiegt. Daher genügen zur Konformationsbeschreibung des Peptidrückgrats in erster Näherung die Torsionswinkel Φ und Ψ der Aminosäurereste. Der Winkel Φ, der die Rotation entlang der N-C_{α}-Bindung beschreibt, wird durch die vier Atome C(=O)-N-C_{α}C(=O) definiert. In gleicherweise definieren N-C_{α}C(=O)-N den Winkel Ψ, der die Rotation um die C_{α}C(=O)-Bindung beschreibt. Obwohl theoretisch eine große Anzahl verschiedener Kombinationen aus Φ und Ψ möglich ist, existieren im allgemeinen in Peptiden bestimmte Vorzugskonformationen in Abhängigkeit von Größe, Polarität und Ladung der Seitenketten, was zur Ausbildung der bekannten Sekundärstrukturen wie α-Helix, ß-Faltblatt, ß-Tum etc. führt.

Aus WO 2008/040332 sind Verbindungen bekannt, die als Mimetika für prolinhaltige Peptide eingesetzt werden können, wenn diese eine Poly-Prolin-Helix (PPII-Helix)-Konformation aufweisen.

Solche prolinhaltigen Prolin-Prolin-Dipeptide, insbesondere solche mit einer PPII-Helix-Konformation, können bevorzugt als Liganden für so genannte PRM-Bindungsdomänen fungieren (PRM = proline-rich motifs).

Prolin als einzige sekundäre Aminosäure der 20 natürlichen Aminosäuren weist durch die Cyclisierung der α-Seitenkette an dem Amidstickstoff einen Torsionswinkel von Φ= (-65 ± 15°) als Bestandteil des fünfgliedrigen Ringes auf und ist damit relativ eingeschränkt; das Peptid hat folglich weniger Rotationsfreiheitsgrade. Die zweifache Alkylierung des Stickstoffs hat einerseits zur Folge, dass das sonst übliche Amidproton (im Peptidrückgrat) fehlt und Prolin deshalb als Wasserstoffbrücken-Donor ausscheidet, andererseits ist die Carbonylgruppe besonders elektronenreich und deshalb ein besserer Wasserstoffbrücken-Akzeptor als bei anderen Aminosäuren. Durch diese geometrischen und elektronischen Eigenschaften kann Prolin keine α-Helix stabilisieren und bildet auch keine ß-Faltblattstruktur, sondern ist in anderen typischen Sekundärstrukturen anzutreffen: den sogenannten ß-Tums und der Poly-Prolin-Helix (PPII-Helix).

Natürliche Peptide liegen jedoch oftmals in einer α-Helix-Konformation vor.

Es besteht daher ein Bedarf an Mimetika, insbesondere an "prolinhaltigen Mimetika" (d.h. Mimetika prolinhaltiger Peptide), die dazu in der Lage sind, die Funktion von Rezeptor-Liganden auszuüben und damit zur Modulation, d.h. zur Stimulation und/oder Hemmung der biologischen Wirkungen eines Peptides und/oder Proteins fähig sind und in einer α-Helix-Konformation vorliegen.

Es war daher eine Aufgabe der vorliegenden Erfindung, Verbindungen bereitzustellen, die die Bildung einer α-Helix-Konformation in solchen Mimetika, vorzugsweise in prolinhaltigen, besonders bevorzugt in prolinreichen Mimetika induzieren, wodurch diese Mimetika zur Modulation, d.h. zur Stimulation und/oder Hemmung der biologischen Wirkungen von in α-Helix-Konformation vorliegenden Peptiden und/oder Proteinen fähig sind, d.h. eine agonistische oder antagonistische Wirkung ausüben.

Diese Aufgabe wird durch den Gegenstand der Patentansprüche gelöst.

Überraschenderweise wurde nun gefunden, dass die substituierten Verbindungen der nachstehend angegebenen allgemeinen Formel (I) die Bildung einer α-Helix-Konformation in Peptiden und/oder Proteinen induzieren und sich daher insbesondere in Arzneimitteln zur Prophylaxe und/oder Behandlung von Störungen oder Krankheiten eignen, welche zumindest teilweise mit der Induktion einer α-Helix-Konformation in wenigstens einem Protein und/oder wenigstens einem Peptid assoziiert sind.

Ein Gegenstand der Erfindung sind substituierte Verbindungen der allgemeinen Formel (I) 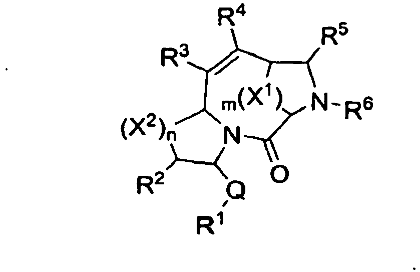 worin
- Q: für C(=O) oder S(=O)₂ steht;
- X¹ und X²: jeweils unabhängig voneinander für O, S, CH₂ oder CHR⁷ stehen,
wobei R⁷ für C₁₋₁₀-Alkyl, gesättigt oder ungesättigt, verzweigt oder unverzweigt, unsubstituiert oder einfach oder mehrfach substituiert, steht;
- m: für 1, 2 oder 3 steht;
- n: für 0, 1, 2 oder 3 steht;
- R¹: für C₁₋₁₀-Alkyl, gesättigt oder ungesättigt, verzweigt oder unverzweigt, unsub-stituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl-verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; OR⁸; NR⁸R⁹; NHC(=O)R⁸; NR⁸R⁹; oder NH-S(=O)₂R⁸; steht,
wobei R⁸ und R⁹ jeweils unabhängig voneinander für H; C₁₋₁₀-Alkyl, gesättigt oder ungesättigt, verzweigt oder unverzweigt, unsubstituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl-verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; stehen;
- oder R¹: für -NHCHR^{a}C(=O)-R^{b} steht, wobei
- R^{a}: ausgewählt ist aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NH-C(=NH)NH₂, -CH₂-Imidazolyl, -CH₂CO₂H, und -CH₂CH₂CO₂H; und
- R^{b}: ausgewählt ist aus -OH und -Peptidyl, wobei das Peptidyl, über seinen N-Terminus kovalent gebunden ist;
- R² und R⁵: jeweils unabhängig voneinander für H; C₁₋₁₀-Alkyl, gesättigt oder ungesättigt, verzweigt oder unverzweigt, unsubstituiert oder einfach oder mehrfach substituiert, stehen;
- R³ und R⁴: jeweils unabhängig voneinander für H; C₁₋₁₀-Alkyl, gesättigt oder ungesättigt, verzweigt oder unverzweigt, unsubstituiert oder einfach oder mehrfach substituiert; Aryl oder Heteroaryl, jeweils unsubstituiert oder einfach oder mehrfach substituiert; stehen;
- R⁶: für H; C₁₋₁₀-Alkyl, gesättigt oder ungesättigt, verzweigt oder unverzweigt, unsubstituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl-verbrücktes C₃₋₁₀-Cycloalkyl oder Heterocyclyl, jeweils gesättigt oder ungesättigt, unsubstituiert oder ein-fach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; C(=O)R¹⁰; C(=O)OR¹⁰; C(=O)NR¹⁰R¹¹; S(=O)₂-R¹⁰; S(=O)₂OR¹⁰; oder S(=O)₂NR¹⁰R¹¹; steht,
wobei R¹⁰ und R¹¹ jeweils unabhängig voneinander für H; C₁₋₁₀-Alkyl, gesättigt oder ungesättigt, verzweigt oder unverzweigt, unsubstituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert, stehen; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; stehen;
oder R⁶ für -C(=O)-CHR^{y}NHR^{z} steht, wobei
- R^{y}: ausgewählt ist aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NH-C(=NH)NH₂, -CH₂-Imidazolyl, -CH₂CO₂H, und -CH₂CH₂CO₂H; und
- R^{z}: ausgewählt ist aus -H und -C(=O)-C₁₋₈-Alkyl;
worin "Alkyl substituiert", "Heterocyclyl substituiert" und "Cycloalkyl substituiert" an den entsprechenden Resten für die Substitution eines oder mehrerer Wasserstoff-atome jeweils unabhängig voneinander durch F; Cl; Br; I; NO₂; CN; CF₃; C₁₋₈-Alkyl; Phenyl ; C(=O)OH; C(=O)O-C₁₋₈-Alkyl; C(=O)O-Phenyl; C(=O)NH₂; C(=O)NH-C₁₋₈-Alky); C(=O)NH-Phenyl; C(=O)N(C₁₋₈-Alkyl)₂; OH; =O; OCF₃; O-C₁₋₈-Alkyl; O-C(=O)-C₁₋₈-Alkyl; SH; SCF₃; S-C₁₋₈-Alkyl; S(=O)₂OH; S(=O)₂C₁₋₈Alkyl; S(=O)₂O-C₁₋₈-Alkyl; S(=O)₂NH-C₁₋₈-Alkyl; S(=O)₂N(C₁₋₈-Alkyl)₂; NH₂; NH-C₁₋₈-Alkyl; NH-Phenyl; N(C₁₋₈-Alkyl)₂; NH-C(=O)-C₁₋₈-Alkyl; NH-S(=O)₂-C₁₋₈-Alkyl, steht;
worin "Aryl substituiert" und "Heteroaryl substituiert" an den entsprechenden Resten für die Substitution eines oder mehrerer Wasserstoffatome jeweils unabhängig voneinander durch F; Cl; Br; I; NO₂; CN; CF₃; C₁₋₈-Alkyl; Phenyl ; C(=O)OH; C(=O)O-C₁₋₈-Alkyl; C(=O)O-Phenyl; C(=O)NH₂; C(=O)NH-C₁₋₈-Alkyl; C(=O)NH-Phenyl; C(=o)N(C₁₋₈-Alkyl)₂; OH; OCF₃; O-C₁₋₈-Alkyl; O-C(=O)-C₁₋₈-Alkyl: SH; SCF₃; S-C₁₋₈-Alkyl; S(=O)₂OH; S(=O)₂C₁₋₈-Alkyl; S(=O)₂O-C₁₋₈-Alkyl; S(=O)₂NH-C₁₋₈-Alkyl; S(=O)₂N(C₁₋₈-Alkyl)₂; NH₂; NH-C₁₋₈-Alkyl; NH-Phenyl; N(C₁₋₈-Alkyl)₂; NH-C(=O)-C₁₋₈-Alkyl; NH-S(=O)₂-C₁₋₈-Alkyl, steht;
jeweils in Form der freien Verbindungen; der Racemate; der Enantiomere, Diastereomere, Mischungen der Enantiomere oder Diastereomere oder eines einzelnen Enantiomers oder Diastereomers; oder in Form der Salze physiologisch verträglicher Säuren oder Basen; oder ggf. in Form von Solvaten.
Unter "Peptidyl" im Sinne der Erfindung ist ein Peptid-Rest zu verstehen, welcher peptidisch verknüpfte proteinogene Aminosäure-Reste umfasst, vorzugsweise der L-Konfiguration, wobei die Gesamtanzahl der Aminosäure-Reste vorzugsweise 1, 2, 3, 4, 5, 6, 7, 8, 9, 10, 11, 12, 13, 14, 15, 16, 17, 18, 19 oder 20 beträgt.

Bevorzugt ist "Peptidyl" folgender Rest:
-NR^{c}'-CHR^{c}-C(=O)-[NR^{d}'-CHR^{d}-C(=O)]_{d}-[NR^{e}'-CHR^{e}-C(=O)]ₑ-[NR^{f}'-CHR^{f}-C(=O)]_{f}-[NR^{g}'-CHR^{g}-C(=O)]_{g}-[NR^{h'}-CHR^{h}-C(=O)]ₕ-[NRⁱ'-CHRⁱ-C(=O)]ᵢ-[NR^{j}'-CHR^{j}-C(=O)]ⱼ-[NR^{k}'-CHR^{k}-C(=O)]ₖ-[NR^{l}'-CHR^{l}-C(=O)]ₗ-[NR^{m}'-CHR^{m}-C(=O)]ₘ-[NRⁿ'-CHRⁿ-C(=O)]ₙ-[NR^{o}'-CHR^{o}-C(=O)]ₒ-[NR^{p}'-CHR^{p}-C(=O)]ₚ-[NR^{q}'-CHR^{q}-C(=O)]_{q}-[NR^{r}'-CHR^{r}-C(=O)]ᵣ-[NR^{s}'-CHR^{s}-C(=O)]ₛ-[NR^{t}'-CHR^{t}-C(=O)]ₜ-[NR^{u}'-CHR^{u}-C(=O)]ᵤ-[NR^{v}'-CHR^{v}-C(=O)]ᵥ-OH,
wobei
R^{c}, R^{d}, R^{e}, R^{f}, R^{g}, R^{h}, Rⁱ, R^{j}, R^{k}, R^{l}, R^{m}, Rⁿ, R^{o}, R^{p}, R^{q}, R^{r}, R^{s}, R^{t}, R^{u} und R^{v} jeweils unabhängig voneinander ausgewählt sind aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NHC(=NH)NH₂, -CH₂-Imidazolyl, -CH₂CO₂H, und -CH₂CH₂CO₂H;
R^{c}', R^{d}', R^{e}', R^{f}', R^{g}', R^{h}', Rⁱ', R^{j}', R^{k}', R^{l}', R^{m}', Rⁿ', R^{o}', R^{p}', R^{q}', R^{r}', R^{s}', R^{t}', R^{u}' und R^{v}' jeweils -H ist;
oder
R^{c} und R^{o}', R^{d} und R^{d}', R^{e} und R^{e}', R^{f} und R^{f}', R^{g} und R^{g}', R^{h} und R^{h}', Rⁱ und Rⁱ', R^{j} und R^{j}', R^{k} und R^{k}', R^{l} und R^{l}', R^{m} und R^{m}', Rⁿ und Rⁿ', R^{o} und R^{o}', R^{p} und R^{p}', R^{q} und R^{q}', R^{r} und R^{r}', R^{s} und R^{s}', R^{t} und R^{t}', R^{u} und R^{u}', oder R^{v} und R^{v}', jeweils gemeinsam -CH₂CH₂CH₂- sind und einen fünfgliedrigen Ring bilden; und
die Indices d, e, f, g, h, i, j, k, l, m, n, o, p, q, r, s, t, u und v jeweils unabhängig voneinander 0 oder 1 sind.

Die Ausdrücke "Alkyl" bzw. "C₁₋₁₀-Alkyl", "C₁₋₈-Alkyl", "C₁₋₄-Alkyl" umfassen im Sinne dieser Erfindung acyclische gesättigte oder ungesättigte aliphatische Kohlenwasser-stoffreste, d.h. C₁₋₁₀-aliphatische Reste, C₁₋₈-aliphatische Reste und C₁₋₄-aliphatische Reste, die jeweils verzweigt oder unverzweigt sowie unsubstituiert oder ein- oder mehrfach substituiert sein können, mit 1 bis 10 bzw. 1 bis 8 bzw. 1 bis 4 Kohlenstoffatomen, d.h. C₁₋₁₀-Alkanyle, C₂₋₁₀-Alkenyle und C₂₋₁₀-Alkinyle bzw. C₁₋₈-Alkanyle, C₂₋₈-Alkenyle und C₂₋₈-Alkinyle bzw. C₁₋₄-Alkanyle, C₂₋₄-Alkenyle und C₂₋₄-Alkinyle. Dabei weisen Alkenyle mindestens eine C-C-Doppelbindung und Alkinyle mindestens eine -C-C-Dreifachbindung auf. Bevorzugt ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, isoPentyl, neo-Pentyl, n-Hexyl, n-Heptyl, n-Octyl, n-Nonyl, n-Decyl, Ethenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), Butenyl, Butinyl, Pentenyl, Pentinyl, Hexenyl und Hexinyl, Heptenyl, Heptinyl, Octenyl, Octinyl, Nonenyl, Noninyl, Decenyl und Decinyl umfasst.

Die Ausdrücke "Cycloalkyl" bzw. "C₃₋₁₀-Cycloalkyl" bedeuten für die Zwecke dieser Erfindung cyclische aliphatische (cycloaliphatische) Kohlenwasserstoffe mit 3, 4, 5, 6, 7, 8, 9 oder 10 Kohlenstoffatomen, d.h. C₃₋₁₀-cycloaliphatische Reste, wobei die Kohlenwasserstoffe gesättigt oder ungesättigt (aber nicht aromatisch), unsubstituiert oder ein- oder mehrfach substituiert sein können. Die Bindung des Cycloalkyls an die jeweilige übergeordnete allgemeine Struktur kann über jedes beliebige und mögliche Ringglied des Cycloalkyl-Restes erfolgen. Die Cycloalkyl-Reste können auch mit weiteren gesättigten, (partiell) ungesättigten, (hetero)cyclischen, aromatischen oder heteroaromatischen Ringsystemen , d.h. mit Cycloalkyl, Heterocyclyl, Aryl oder Heteroaryl kondensiert sein, die wiederum unsubstituiert oder einfach oder mehrfach substituiert sein können. Die Cycloalkyl-Reste können weiterhin einfach oder mehrfach verbrückt sein wie bspw. im Fall von Adamantyl, Bicyclo[2.2.1]heptyl oder Bicyclo[2.2.2]octyl. Bevorzugt ist Cycloalkyl aus der Gruppe ausgewählt, die Cyclopropyl, Cyclobutyl, Cyclopentyl, Cyclohexyl, Cycloheptyl, Cyclooctyl,
Cyclononyl, Cyclodecyl, Adamantyl, 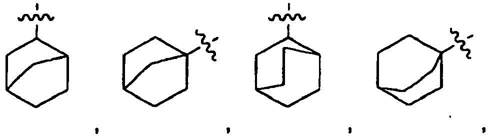 Cyclopentenyl, Cyclohexenyl, Cycloheptenyl und Cyclooctenyl umfasst.

Die Begriffe "Heterocyclyl" bzw. "Heterocycloalkyl" umfassen aliphatische gesättigte oder ungesättigte (aber nicht aromatische) Cycloalkyle mit drei bis zehn, d.h. 3, 4, 5, 6, 7, 8, 9 oder 10 Ringgliedern, in denen mindestens ein, ggf. auch zwei oder drei Kohlenstoffatome durch ein Heteroatom oder eine Heteroatomgruppe jeweils unabhängig voneinander ausgewählt aus der Gruppe bestehend aus O, S, S(=O)₂, N, NH und N(C₁₋₈-Alkyl), vorzugsweise N(CH₃) ersetzt sind, wobei die Ringglieder unsubstituiert oder ein- oder mehrfach substituiert sein können. Heterocycle sind damit heterocycloaliphatische Reste. Die Bindung des Heterocyclyls an die übergeordnete allgemeine Struktur kann über jedes beliebige und mögliche Ringglied des Heterocyclyl-Restes erfolgen. Die Heterocyclyl-Reste können auch mit weiteren gesättigten, (partiell) ungesättigten (hetero)cyclischen oder aromatischen oder heteroaromatischen Ringsystemen, d.h. mit Cycloalkyl, Heterocyclyl, Aryl oder Heteroaryl kondensiert sein, die wiederum unsubstituiert oder einfach oder mehrfach substituiert sein können. Bevorzugt sind Heterocyclyl-Reste aus der Gruppe umfassend Azetidinyl, Aziridinyl, Azepanyl, Azocanyl, Diazepanyl, Dithiolanyl, Dihydrochinolinyl, Dihydropyrrolyl, Dioxanyl, Dioxolanyl, Dioxepanyl, Dihydroindenyl Dihydropyridinyl, Dihydrofuranyl, Dihydroisochinolinyl, Dihydroindolinyl, Dihydroisoindolyl, Imidazolidinyl, Isoxazolidinyl, Morpholinyl, Oxiranyl, Oxetanyl, Pyrrolidinyl, Piperazinyl, 4-Methylpiperazinyl, Piperidinyl, Pyrazolidinyl, Pyranyl, Tetrahydro-pyrrolyl, Tetrahydropyranyl, Tetrahydrochinolinyl, Tetrahydroisochinolinyl, Tetrahydroindolinyl, Tetrahydrofuranyl, Tetrahydropyridinyl, Tetrahydrothiophenyl, Tetrahydro-pyridoindolyl, Tetrahydronaphthyl, Tetrahydrocarbolinyl, Tetrahydroisoxazolo-pyridinyl, Thiazolidinyl und Thiomorpholinyl.

Der Begriff "Aryl" bedeutet im Sinne dieser Erfindung aromatische Kohlenwasser-stoffe mit bis zu 14 Ringgliedern, u.a. Phenyle und Naphthyle. Jeder Aryl-Rest kann unsubstituiert oder einfach oder mehrfach substituiert vorliegen, wobei die ArylSubstituenten gleich oder verschieden und in jeder beliebigen und möglichen Position des Aryls sein können. Die Bindung des Aryls an die übergeordnete allgemeine Struktur kann über jedes beliebige und mögliche Ringglied des ArylRestes erfolgen. Die Aryl-Reste können auch mit weiteren gesättigten, (partiell) ungesättigten, (hetero)cyclischen, aromatischen oder heteroaromatischen Ringsystemen kondensiert sein, d.h. mit Cycloalkyl, Heterocyclyl, Aryl oder Heteroaryl, die wiederum unsubstituiert oder einfach oder mehrfach substituiert sein können. Ein Beispiel für einen kondensierten Aryl-Rest ist Fluorenyl. Bevorzugt ist Aryl aus der Gruppe ausgewählt, die Phenyl, Fluorenyl, 1-Naphthyl und 2-Naphthyl enthält, welche jeweils unsubstituiert oder ein- oder mehrfach substituiert sein können. Ein besonders bevorzugtes Aryl ist Phenyl, unsubstituiert oder einfach oder mehrfach substituiert.

Der Begriff "Heteroaryl" steht für einen 5- oder 6-gliedrigen cyclischen aromatischen Rest, der mindestens 1, ggf. auch 2, 3, 4 oder 5 Heteroatome, enthält, wobei die Heteroatome jeweils unabhängig voneinander ausgewählt sind aus der Gruppe S, N und O und der Heteroaryl-Rest unsubstituiert oder ein- oder mehrfach substituiert sein kann; im Falle der Substitution am Heteroaryl können die Substituenten gleich oder verschieden sein und in jeder beliebigen und möglichen Position des Heteroaryls sein. Die Bindung an die übergeordnete allgemeine Struktur kann über jedes beliebige und mögliche Ringglied des Heteroaryl-Restes erfolgen. Das Heteroaryl kann auch Teil eines bi- oder polycyclischen Systems mit bis zu 14 Ringgliedern sein, wobei das Ringsystem mit weiteren gesättigten, (partiell) ungesättigten, (hetero)cyclischen oder aromatischen oder heteroaromatischen Ringen gebildet werden kann, d.h. mit Cycloalkyl, Heterocyclyl, Aryl oder Heteroaryl, die wiederum unsubstituiert oder einfach oder mehrfach substituiert sein können. Es ist bevorzugt, dass der Heteroaryl-Rest ausgewählt ist aus der Gruppe, die Benzofuranyl, Benzoimidazolyl, Benzothienyl, Benzothiadiazolyl, Benzothiazolyl, Benzotriazolyl, Benzooxazolyl, Benzooxadiazolyl, Chinazolinyl, Chinoxalinyl, Carbazolyl, Chinolinyl, Dibenzofuranyl, Dibenzothienyl, Furyl (Furanyl), Imidazolyl, Imidazothiazolyl, Indazolyl, Indolizinyl, Indolyl, Isochinolinyl, Isoxazoyl, Isothiazolyl, Indolyl, Naphthyridinyl, Oxazolyl, Oxadiazolyl, Phenazinyl, Phenothiazinyl, Phtalazinyl, Pyrazolyl, Pyridyl (2-Pyridyl, 3-Pyridyl, 4-Pyridyl), Pyrrolyl, Pyridazinyl, Pyrimidinyl, Pyrazinyl, Purinyl, Phenazinyl, Tetrazol, Thienyl (Thiophenyl), Triazolyl, Tetrazolyl, Thiazolyl, Thiadiazolyl oder Triazinyl umfässt. Besonders bevorzugt sind Pyridyl und Thienyl.

Die Ausdrücke "über C₁₋₄-Alkyl oder C₁₋₈-Alkyl verbrücktes Aryl, Heteroaryl, Heterocyclyl oder Cycloalkyl" bedeuten im Sinne der Erfindung, dass C₁₋₄-Alkyl oder C₁₋₈-Alkyl und Aryl bzw. Heteroaryl bzw. Heterocyclyl bzw. Cycloalkyl die oben definierten Bedeutungen haben und der Aryl- bzw. Heteroaryl- bzw. Heterocyclyl- bzw. Cyclo-alkyl-Rest über eine C₁₋₄-Alkyl- oder eine C₁₋₈-Alkyl-Gruppe an die jeweilige übergeordnete allgemeine Struktur gebunden ist. Die Alkylkette der Alkyl-Gruppe kann in allen Fällen verzweigt oder unverzweigt, unsubstituiert oder einfach oder mehrfach substituiert sein. Die Alkylkette der Alkyl-Gruppe kann ferner in allen Fällen gesättigt oder ungesättigt sein, d.h. kann eine Alkylengruppe, d.h. eine C₁₋₄-Alkylengruppe oder eine C₁₋₈-Alkylengruppe, eine Alkenylengruppe, d.h. eine C₂₋₄-Alkenylengruppe oder eine C₂₋₈-Alkenylengruppe, oder eine Alkinylengruppe, d.h. eine G₂₋₄-Alkinylengruppe oder eine C₂₋₈-Alkinylengruppe sein. Vorzugsweise ist C₁₋₄-Alkyl ausgewählt aus der Gruppe umfassend -CH₂-, -CH₂-CH₂-, -CH(CH₃)-, -CH₂-CH₂-CH₂-, -CH(CH₃)-CH₂-, -CH(CH₂CH₃)-, -CH₂-(CH₂)₂-CH₂-, -CH(CH₃)-CH₂-CH₂-, -CH₂-CH(CH₃)-CH₂-, -CH(CH₃)-CH(CH₃)-, -CH(CH₂CH₃)-CH₂-, -C(CH₃)₂-CH₂-, -CH(CH₂CH₂CH₃)-, -C(CH₃)(CH₂CH₃)-, -CH=CH-, -CH=CH-CH₂-, -C(CH₃)=CH₂-, -CH=CH-CH₂-CH₂-, -CH₂-CH=CH-CH₂-, -CH=CH-CH=CH-, -C(CH₃)=CH-CH₂-, -CH=C(CH₃)-CH₂-, -C(CH₃)=C(CH₃)-, -C(CH₂CH₃)=CH-, -C=C-, -C≡C-CH₂-, -C=C-CH₂-CH₂-, -C≡C-CH(CH₃)-, -CH₂-C≡C-CH₂- und -C≡C-C≡C- und C₁₋₈-Alkyl ausgewählt aus der Gruppe umfassend -CH₂-, -CH₂-CH₂-, -CH(CH₃)-, -CH₂-CH₂-CH₂-, -CH(CH₃)-CH₂-, -CH(CH₂CH₃)-, -CH₂-(CH₂)₂-CH₂-, -CH(CH₃)-CH₂-CH₂-, -CH₂-CH(CH₃)-CH₂-, -CH(CH₃)-CH(CH₃)-, -CH(CH₂CH₃)-CH₂-, -C(CH₃)₂-CH₂-, -CH(CH₂CH₂CH₃)-, -C(CH₃)(CH₂CH₃)-, -CH₂-(CH₂)₃-CH₂-, -CH(CH₃)-CH₂-CH₂-CH₂-, -CH₂-CH(CH₃)-CH₂-CH₂-, -CH(CH₃)-CH₂-CH(CH₃)-, -CH(CH₃)-CH(CH₃)-CH₂-, -C(CH₃)₂-CH₂-CH₂-, -CH₂-C(CH₃)₂-CH₂-, -CH(CH₂CH₃)-CH₂-CH₂-, -CH₂-CH(CH₂CH₃)-CH₂-, -C(CH₃)₂-CH(CH₃)-, -CH(CH₂CH₃)-CH(CH₃)-, -C(CH₃)(CH₂CH₃)-CH₂-, -CH(CH₂CH₂CH₃)-CH₂-, -C(CH₂CH₂CH₃)-CH₂-, -CH(CH₂CH₂CH₂CH₃)-, -C(CH₃)(CH₂CH₂CH₃)-, -C(CH₂CH₃)₂-, -CH₂-(CH₂)₄-CH₂-, -CH=CH-, -CH=CH-CH₂-, -C(CH₃)=CH₂-, -CH=CH-CH₂-CH₂-, -CH₂-CH=CH-CH₂-, -CH=CH-CH=CH-, -C(CH₃)=CH-CH₂-, -CH=C(CH₃)-CH₂-, -C(CH₃)=C(CH₃)-, -C(CH₂CH₃)=CH-, -CH=CH-CH₂-CH₂-CH₂-, -CH₂-CH=CH₂-CH₂-CH₂-, -CH=CH=CH-CH₂-CH₂-, -CH=CH₂-CH-CH=CH₂-, -C≡C-, -C≡C-CH₂-, -C≡C-CH₂-CH₂-, -C≡C-CH(CH₃)-, -CH₂-C≡C-CH₂-, -C≡C-C≡C-, -C≡C-C(CH₃)₂-, -C≡C-CH₂-CH₂-CH₂-, -CH₂-C≡C-CH₂-CH₂-, -C≡C-C≡C-CH₂- und -C≡C-CH₂-C≡C-,

Im Zusammenhang mit "Alkyl", "Heterocyclyl" und "Cycloalkyl" versteht man unter dem Begriff "ein- oder mehrfach substituiert" im Sinne dieser Erfindung die ein- oder mehrfache, z.B. zwei-, drei- oder vierfache Substitution eines oder mehrerer Wasserstoffatome jeweils unabhängig voneinander durch Substituenten ausgewählt aus der Gruppe aus F; Cl; Br; I; NO₂; CN; CF₃; C₁₋₈-Alkyl; Phenyl ; C(=O)OH; C(=O)O-C₁₋₈-Alkyl; C(=O)O-Phenyl; C(=O)NH₂; C(=O)NH-C₁₋₈-Alkyl; C(=O)NH-Phenyl; C(=O)N(C₁₋₈-Alkyl)₂; OH; =O; OCF₃; O-C₁₋₈-Alkyl; O-C(=O)-C₁₋₈-Alkyl; SH; SCF₃; S-C₁₋₈-Alkyl; S(=O)₂OH; S(=O)₂C₁₋₈-Alkyl; S(=O)₂O-C₁₋₈-Alkyl; S(=O)₂NH-C₁₋₈-Alkyl; S(=O)₂N(C₁₋₈-Alkyl)₂; NH₂; NH-C₁₋₈-Alkyl; NH-Phenyl; N(C₁₋₈-Alkyl)₂, NH-C(=O)-C₁₋₈-Alkyl; NH-S(=O)₂-C₁₋₈-Alkyl; wobei unter mehrfach substituierten Resten solche Reste zu verstehen sind, die entweder an verschiedenen oder an gleichen Atomen mehrfach, z.B. zwei-, drei- oder vierfach substituiert sind, beispielsweise dreifach am gleichen C-Atom wie im Falle von CF₃ oder CH₂CF₃ oder an verschiedenen Stellen wie im Falle von CH(OH)-CH=CH-CHCl₂. Ein Substituent kann gegebenenfalls seinerseits wiederum einfach oder mehrfach substituiert sein. Die Mehrfachsubstitution kann mit dem gleichen oder mit verschiedenen Substituenten erfolgen.

Bevorzugte "Alkyl-", "Heterocyclyl-" und "Cycloalkyl-" Substituenten sind ausgewählt aus der Gruppe aus F, Cl, Br, I, C₁₋₄-Alkyl, C(O)OH, C(=O)OC₁₋₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄Alkyl, NHS(=O)₂C₁₋₄-Alkyl, S(=O)₂OH, S(=O)₂OC₁₋₄-Alkyl und S(=O)₂C₁₋₄-Alkyl.

Im Zusammenhang mit "Aryl" und "Heteroaryl" versteht man im Sinne dieser Erfindung unter "ein- oder mehrfach substituiert" die ein- oder mehrfache, z.B. zwei-, drei- oder vierfache Substitution eines oder mehrerer Wasserstoffatome des Ringsystems jeweils unabhängig voneinander durch Substituenten ausgewählt aus der Gruppe aus F; Cl; Br; I; NO₂; CN; CF₃; C₁₋₈-Alkyl; Phenyl; C(=O)OH; C(=O)O-C₁₋₈-Alkyl; C(=O)O-Phenyl; C(=O)NH₂; C(=O)NH-C₁₋₈Alkyl; C(=O)NH-Phenyl; C(=O)N(C₁₋₆-Alkyl)₂; OH; OCF₃; O-C₁₋₈-Alkyl; O-C(O)-C₁₋₈-Alkyl; SH; SCF₃; S-C₁₋₈-Alkyl; S(=O)₂OH; S(=O)₂C₁₋₈-Alkyl; S(=O)₂O-C₁₋₈-Alkyl; S(=O)₂NH-C₁₋₈-Alkyl; S(=O)₂N(C₁₋₈-Alkyl)₂; NH₂; NH-C₁₋₈-Alkyl; NH-Phenyl; N(C₁₋₈-Alkyl)₂: NH-C(=O)-C₁₋₈-Alkyl; NH-S(=O)₂-C₁₋₈-Alkyl; an einem oder ggf. verschiedenen Atomen, wobei ein Substituent ggf. seinerseits wiederum einfach oder mehrfach substituiert sein kann. Die Mehrfachsubstitution erfolgt mit dem gleichen oder mit unterschiedlichen Substituenten.

Bevorzugte "Aryl-" und "Heteroaryl-" Substituenten sind ausgewählt aus der Gruppe bestehend aus F, Cl, Br, I, C₁₋₄-Alkyl, C(=O)CH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl.

Die Verbindungen der allgemeinen Formel (I) sind durch Substituenten definiert, beispielsweise durch R¹, R² und H³ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Generation). Ist beispielsweise R¹ = Aryl (Substituent der 1. Generation), so kann Aryl seinerseits substituiert sein, z.B. mit C₁₋₈-Alkyl (Substituent der 2. Generation). Es ergibt sich daraus die funktionelle Gruppe Aryl-C₁₋₈-Alkyl. C₁₋₈-Alkyl kann dann seinerseits erneut substituiert sein, z.B. mit Cl (Substituent der 3. Generation). Es ergibt sich daraus dann insgesamt die funktionelle Gruppe Aryl-C₁₋₈-Alkyl-Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform bspw. im Fall der allgemeinen Formel (I) die funktionellen Gruppen für R¹ bis R¹¹ jeweils ggf. substituiert sein, die jeweiligen Substituenten können dann ihrerseits jedoch nicht erneut substituiert sein.

Im Rahmen der vorliegenden Erfindung bezeichnet das in Formeln verwendete Symbol 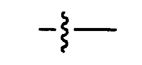 eine Verknüpfung eines entsprechenden Restes an die jeweilige übergeordnete allgemeine Struktur.

Wenn ein Rest innerhalb eines Moleküls mehrfach vorkommt, wie z.B. der Rest R⁷, dann kann dieser Rest für verschiedene Substituenten jeweils unterschiedliche Bedeutungen haben: sind beispielsweise sowohl X¹ = CHR⁷ als auch X² = CHR⁷, so kann R⁷ für X¹ = H und R⁷ für X² = C₁₋₁₀-Alkyl bedeuten.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevor-zugt ist das Hydrochlorid. Beispiele für physiologisch verträgliche Säuren sind: Salzsäure, Bromwasserstoffsäure, Schwefelsäure, Methansulfonsäure, p-Toluol-sulfonsäure, Kohlensäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Hippursäure, Phosphorsäure, Asparaginsäure. Besonders bevorzugt sind die Zitronensäure und die Salzsäure.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetierverträglich sind. Besonders bevorzugt sind die Salze der Alkali- und Erdalkalimetalle aber auch Ammoniumsalze (NHₓR₄₋ₓ]⁺, worin x = 0, 1, 2, 3 oder 4 ist und R für einen verzweigten oder unverzweigten, gesättigten oder ungesättigten C₁₋₁₀-Alkyl-Rest, unsubstituiert oder einfach oder mehrfach substituiert oder für einen C₃₋₁₀-Cycloalkyl-Rest, gesättigt oder ungesättigt, unsubstituiert oder einfach oder mehrfach substituiert steht oder zwei der Reste R gemeinsam mit dem Stickstoffatom ein Heterocyclyl, gesättigt oder ungesättigt, unsubstituiert oder einfach oder mehrfach substituiert, vorzugsweise ein Pyrrolidinyl, Piperidinyl oder Morpholinyl bilden. Ganz besonders bevorzugt sind (Mono-) oder (Di-) Lithium-, (Mono-) oder (Di-) Natrium-, (Mono-) oder (Di-) Kalium-, Magnesium- oder Calcium-Salze.

Bevorzugte Ausführungsform der Verbindungen der allgemeinen Formel (I) haben die allgemeine Formel (Ia), (Ib) und (Ic)

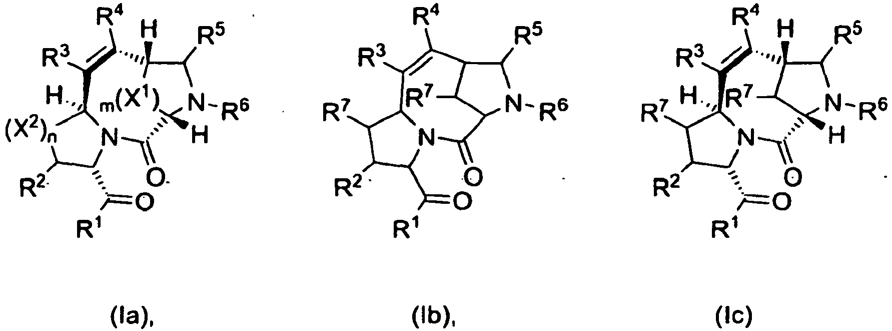

In einer bevorzugten Ausführungsform der erfindungsgemäßen Verbindungen der allgemeinen Formel (I) stehen
- X¹ und X²: jeweils für CH₂.
- m: für 1 oder 2, vorzugsweise für 1;
- n: für 0, 1 oder 2, vorzugsweise für 1.

Ein Fachmann erkennt, dass sich die Struktur der Verbindungen gemäß der allgemeinen Formeln (Ia), (Ib) und (Ic) von Prolin bzw. Diprolin ableitet. Vorzugsweise ist die Stereochemie derart, dass sie sich von der L-Konfiguration des Prolins bzw. Diprolins ableitet.

In einer weiteren bevorzugten Ausführungsform der Verbindungen der allgemeinen Formel (I) steht
- R¹: für OR⁸; NHR⁸, R⁸ oder NHC(=O)R⁸; vorzugsweise für OR⁸,
wobei R⁸ für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, C(=O)OH, C(=O)OC₁₋₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl; Aryl, unsubstituiert oder einfach oder mehrfach substituiert mit einem oder mehreren Substituenten jeweils unabhängig voneinander ausgewählt aus der Gruppe bestehend aus F, Cl. Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, S(=O)₂OH, S(=)₂OC₁₋₄-Alkyl und S(=O)₂C₁₋₄-Alkyl; oder über C₁₋₈-Alkyl-verbrücktes Aryl, unsubstituiert oder einfach oder mehrfach substituiert mit einem oder mehreren Substituenten jeweils unabhängig voneinander ausgewählt aus der Gruppe bestehend aus F, CI, Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl; wobei die Alkylkette jeweils verzweigt oder unverzweigt, gesättigt oder ungesättigt, unsubstituiert sein kann; steht;

- oder R¹: steht für -NHCHR^{a}C(=O)-R^{b},
wobei R^{a} ausgewählt ist aus der Gruppe bestehend aus -H, -CH₃, -CH(CH₃)₂,-CH₂CH(CH₃)₂, -CH(CH₃)CH₂CH₃, -CH₂CH₂SCH₃, -CH₂C₆H,₅, -CH₂-Indolyl,-CH₂OH, -CH(OH)CH₃, -CH₂C(=O)NH₂, CH₂CH₂C(=O)NH₂, -CH₂-C₆H₄-OH,-CH₂SH, -CH₂CH₂CH₂CH₂NH₂, -CH₂CH₂CH₂NHC(=NH)NH₂, -CH₂-Imidazolyl,-CH₂CO₂H, und -CH₂CH₂CO₂H; und
- R^{b}: ausgewählt ist aus -OH und -Peptidyl, wobei das Peptidyl, über seinen N-Terminus kovalent gebunden ist.

Vorzugsweise steht R¹ für OH
- oder R¹: steht für -NHCHR^{a}C(=O)-R^{b},
wobei R^{a} ausgewählt ist aus der Gruppe bestehend aus -H, -CH₃, -CH(CH₃)₂,-CH₂CH(CH₃)₂, -CH(CH₃)CH₂CH₃, -CH₂CH₂SCH₃, -CH₂C₆H₅, -CH₂-Indolyl,-CH₂OH, -CH(OH)CH₃, -CH₂C(=O)NH₂, CH₂CH₂C(=O)NH₂, -CH₂-C₆H₄-OH,-CH₂SH, -CH₂CH₂CH₂CH₂NH₂, -CH₂CH₂CH₂NHC(=NH)NH₂, -CH₂-Imidazolyl,-CH₂CO₂H, und -CH₂CH₂CO₂H; und
- R^{b}: ausgewählt ist aus -OH und -Peptidyl, wobei das Peptidyl, über seinen N-Terminus kovalent gebunden ist.

Besonders bevorzugt steht R¹ für OH oder für -NHCH₂C(=O)-Peptidyl.

In einer besonders bevorzugten Ausführungsform steht R¹ für OH.

In einer anderen einer besonders bevorzugten Ausführungsform steht R¹ für-NHCH₂C(=O)-Peptidyl.

In einer weiteren bevorzugten Ausführungsform der Verbindungen der allgemeinen Formel (I) stehen
- R² und R⁵: jeweils unabhängig voneinander für H; C₁₋₁₀-Alkyl, gesättigt oder ungesättigt, verzweigt oder unverzweigt, unsubstituiert, und
- R³ und R⁴: jeweils unabhängig voneinander für H; C₁₋₁₀-Alkyl, gesättigt oder ungesättigt, verzweigt oder unverzweigt, unsubstituiert.

Vorzugsweise stehen R², R³, R⁴ und R⁵ jeweils für H.

In einer weiteren bevorzugten Ausführungsform der Verbindungen der allgemeinen Formel (I) steht
- R⁶: für H; C₁₋₁₀-Alkyl, gesättigt oder ungesättigt, verzweigt oder unverzweigt, substituiert mit einem oder mehreren Substituenten jeweils unabhängig voneinander ausgewählt aus der Gruppe bestehend aus F, Cl, Br, I, C(=O)OH, C(=O)OC₁₋₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl;
oder für C(=O)R¹⁰; C(=O)OR¹⁰; C(=O)NR¹⁰R¹¹; S(=O)₂-R¹⁰; S(=O)₂OR¹⁰; oder S(=O)₂NR¹⁰R¹¹,
wobei R¹⁰ und R¹¹ jeweils unabhängig voneinander für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl, stehen; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl; wobei die Alkylkette jeweils verzweigt oder unverzweigt, gesättigt oder ungesättigt, unsubstituiert sein kann; stehen;
oder R⁶ steht für -C(=O)-CHR^{y}NHR^{z}, wobei
- R^{y}: ausgewählt ist aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NH-C(=NH)NH₂, -CH₂-Imidazolyl, -CH₂CO₂H, und -CH₂CH₂CO₂H; und
- R^{z}: ausgewählt ist aus -H und -C(=O)-C₁₋₈-Alkyl.

### Vorzugsweise steht

R⁶ für C(=O)R¹⁰ oder C(=O)OR¹⁰, wobei R¹⁰ für über C₁₋₈-Alkyl-verbrücktes Aryl, unsubstituiert, besonders bevorzugt für über C₁₋₈-Alkyl-verbrücktes Fluorenyl steht,
oder R⁶ steht für -C(=O)-CHR^{y}NHR^{z}, wobei
R^{y} ausgewählt ist aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NHC(=NH)NH₂, -CH₂-Imidazolyl, -CH₂CO₂H, und -CH₂CH₂CO₂H; und
R^{z} ausgewählt ist aus -H und -C(=O)-C₁₋₈-Alkyl.

Besonders bevorzugt steht R⁶ für eine Fmoc-Gruppe (Fluorenylmethoxycarbonyl-Gruppe) oder R⁶ steht für -C(=O)-CH₂NHC(=O)CH₃.

In einer besonders bevorzugten Ausführungsform steht R⁶ für eine Fmoc-Gruppe.

In einer anderen besonders bevorzugten Ausführungsform steht R⁶ für -C(=O)-CH₂NH-C(=O)CH₃.

In einer besonders bevorzugten Ausführungsform der Verbindungen der allgemeinen Formel (I) stehen
- X¹ und X²: jeweils für CH₂,
- m und n: jeweils für 1,
- R¹: für OR⁸,
wobei R⁸ für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C(=O)OH, C(=O)OC₁₋₄-Alkyl, C(=O)C₁₋₄-Alkyl,C(=O)NH-C₁₋₄-Alkyl, OH, O-C₁₋₄-Alkyl, =O, NH₂, NHC(=O)C₁₋₄-Alkyl,NHS(=O)₂C₁₋₄-Alkyl, S(=O)₂OH, S(=O)₂OC₁₋₄-Alkyl und S(=O)₂C₁₋₄-Alkyl; oderüber C₁₋₈-Alkyl-verbrücktes Aryl, unsubstituiert oder einfach oder mehrfachsubstituiert mit einem oder mehreren Substituenten jeweils unabhängigvoneinander ausgewählt aus der Gruppe bestehend aus F, Cl, Br, I, C₁₋₄-Alkyl,C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, S(=O)₂OH, S(=O)₂OC₁₋₄-Alkyl, und S(=O)₂C₁₋₄-Alkyl; wobei die Alkylkette jeweils verzweigt oderunverzweigt, gesättigt oder ungesättigt, unsubstituiert sein kann; steht;
- oder R¹: steht für -NHCHR^{a}C(=O)-R^{b},
wobei R^{a} ausgewählt ist aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NHC(=NH)NH₂, -CH₂-Imidazolyl, -CH₂CO₂H, und -CH₂CH₂CO₂H; und
R^{b} ausgewählt ist aus -OH und -Peptidyl, wobei das Peptidyl, über seinen N-Terminus kovalent gebunden ist;
- R² und R⁵: jeweils für H;
- R³ und R⁴: jeweils für H;
- R⁶: für H; C₁₋₁₀-Alkyl, gesättigt oder ungesättigt, verzweigt oder unverzweigt, substituiert mit einem oder mehreren Substituenten jeweils unabhängig voneinander ausgewählt aus der Gruppe bestehend aus C(=O)OH, C(=O)OC₁₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄-Alkyl und NHS(=O)₂C₁₋₄-Alkyl;
oder für C(=O)R¹⁰; C(=O)OR¹⁰; C(=O)NR¹⁰R¹¹; S(=O)₂-R¹⁰; S(=O)₂OR¹⁰; oder S(=O)₂NR¹⁰R¹¹.
wobei R¹⁰ und R¹¹ jeweils unabhängig voneinander für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄-Alkyl, NHS(=O)₂C₁₋₄-Alkyl; Aryl, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, stehen; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, wobei die Alkylkette jeweils verzweigt oder unverzweigt, gesättigt oder ungesättigt, unsubstituiert sein kann; stehen,
oder R⁶ steht für -C(=O)-CHR^{y}NHR^{z}, wobei
- R^{y}: ausgewählt ist aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NHC(=NH)NH₂, -CH₂-Imidazolyl, -CH₂CO₂H, und -CH₂CH₂CO₂H; und
- R^{z}: ausgewählt ist aus -H und -C(=O)-C₁₋₈-Alkyl.

Ein weiterer Gegenstand der vorliegenden Erfindung ist die Verwendung wenigstens einer Verbindung der allgemeinen Formel (I) für die Induktion einer α-Helix-Konformation in wenigstens einem Protein und/oder Peptid.

Ein weiterer Gegenstand der vorliegenden Erfindung ist ein Arzneimittel enthaltend wenigstens eine erfindungsgemäße Verbindung der vorstehend angegebenen allgemeinen Formel (I), welche vorzugsweise eine α-Helix-Konformation in wenigstens einem Protein und/oder Peptid induziert, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie gegebenenfalls einen oder mehrere pharmazeutisch verträgliche Hilfsstoffe und/oder gegebenenfalls weitere Wirkstoffe.

Bevorzugte pharmazeutisch verträgliche Hilfsstoffe sind beispielsweise Füllmittel, Streckmittel, Bindemittel, Feuchthaltemittel, Lösungsverzögerer, Sprengmittel, Resorptionsbeschleuniger, Netzmittel, Absorptionsmittel und/oder Gleitmittel.

In einer bevorzugten Ausführungsform enthält das erfindungsgemäße Arzneimittel wenigstens ein Peptid und/oder Protein, welches eine α-Helix-Konformation aufweist, die durch wenigstens eine in dem erfindungsgemäßen Arzneimittel enthaltene erfindungsgemäße substituierte Verbindung der allgemeinen Formel (I) induziert worden ist.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpresst, in Kapseln abgefüllt oder in einer Flüssigkeit suspendiert, vorliegen und als solche auch verabreicht werden.

Diese erfindungsgemäßen Arzneimittel eignen sich insbesondere zur Induktion der Bildung einer α-Helix-Konformation in Peptiden und/oder Proteinen eignen und sich daher insbesondere in Arzneimitteln zur Prophylaxe und/oder Behandlung von Störungen oder Krankheiten eignen, welche zumindest teilweise mit der Induktion einer α-Helix-Konformation in wenigstens einem Protein und/oder wenigstens einem Peptid assoziiert sind.

Ein weiterer Gegenstand der vorliegenden Erfindung ist daher die Verwendung wenigstens einer substituierten Verbindung der allgemeinen Formel (I) 6 zur Herstellung eines Arzneimittels zur Behandlung und/oder Prophylaxe von einer oder mehrerer Erkrankungen und/oder Störungen, welche zumindest teilweise mit der Induktion einer α-Helix-Konformation in wenigstens einem Protein und/oder wenigstens einem Peptid assoziiert sind.

Bevorzugt eignet sich das erfindungsgemäße Arzneimittel zur Behandlung und/oder Prophylaxe von einer oder mehreren Erkrankungen und/oder Störungen ausgewählt aus der Gruppe bestehend aus bakterielle und virale Infektionskrankeiten, neurodegenerative Erkrankungen und Tumorerkrankungen.

Weitere Krankheiten im Sinne der Erfindung, die mit den erfindungsgemäßen Amzeimitteln behandelt werden können, sind ausgewählt aus der Gruppe umfassend Affenpocken, AIDS, Anthrax (Bacillus anthracis, Milzbrand), Aviäre Influenza (Geflügelpest, Vogelgrippe), Borreliose, Borrelia recurrentis (Läuserückfallfieber), Botulismus (Clostridium botulinum), Brucellose, Campylobacter-Infektionen, Chlamydiose, Cholera (Vibrio cholerae), CreutzfeldWakob-Krankheit, Coxiella bumetii (Q-Fieber), Cryptosporidium parvuum (Kryptosporidiose), Dengue-Fieber, Diphtherie, Ebolavirus-Infektionen, Echinokokkose (Fuchsbandwurm, Hundebandwurm), EHEC-Infektionen (STEC-Infektionen, VTEC-Infektionen), Enteroviren, Fleckfieber (Rickettsia prowazeckii), Francisella tularensis (Tularämie), Frühsommer Meningoenzephalitis (FSME), Gelbfieber, Giardiasis, Gonorrhoe, Grippe (Influenza), Haemophilis influenzae, Hantavirus, Helicobacter pylori, Hepatitis A, Hepatitis B, Hepatitis C, Hepatitis D, Hepatitis E, Herpes, HUS (hämolytisch urämisches Syndrom), Keratoconjunctivitis epidemica, Keuchhusten (Pertussis), Kinderlähmung (Poliomyelitis), Kopflausbefall, Krätzemilbenbefall, Krim-Kongo-Fieber, LassaFieber, lebensmittelbedingte Krankheiten, Legionellose, Leishmaniose, Lepra, Leptospirose, Listeriose, Lyme-Borreliose, Lymphogranuloma venereum, Malaria (Plasmodien-Infektionen), MarburgvirusInfektionen, Masern, Meliodose, Meningokokken-Erkrankungen, MRSA (Staphylokokken), Mumps, Mykosen (Pilzinfektionen), neu und vermehrt auftretende Infektionskrankheiten, Noroviren, Omithose (Papageienkrankheit), Papillomaviren, Paratyphus, Pest (Yersinia pestis), Pneumokokken-Infektionen (Streptococcus pneumoniae), Pocken, reiseassoziierte Infektionskrankheiten, RinderbandwurmInfektion beim Menschen, Rotaviren, Röteln, RSV-Infektionen, Salmonellose, Scharlach, Schweres Akutes Respiratorisches Syndrom (SARS), sexuell übertragbare Infektionen, Shigellose, Syphilis, Tetanus, Tollwut, Toxoplasmose, Thrichinellose, Tuberkulose, Typhus, Varizellen (Windpocken), Variante Creutzfeldt-Jakob-Krankheit, virale hämorrhagische Fieber, West-Nil-Fieber, Yersiniose und/oder Zecken-übertragene Erkrankungen.

Bevorzugt handelt es sich bei den bakteriellen Erkrankungen um Erkrankungen, die durch folgende Bakterien assoziiert, insbesondere vermittelt werden: Legionellen, Streptokokken, Staphylokokken, Klebsiellen, Haemophilis influenzae, Rickettsien (Fleckfieber), Mykobakterien, Mykoplasmen, Ureaplasmen, Neisserien (Meningitis, Waterhouse-Friedrichsen-Syndrom, Gonorrhoe), Pseudomonaden, Bordetellen (Pertussis), Corynobakterien (Diphtherie), Chlamydien, Campylobacter (Durchfall), Escherichia coli, Proteus, Salmonellen, Shigellen, Yersinien, Vibrionen, Enterokokken, Clostridien, Borrelien, Treponema pallidum, Brucellen, Francisellen und/oder Leptospira, insbesondere Listerien.

Besonders bevorzugte Erkrankungen sind die, die durch Listerien ausgewählt aus der Gruppe umfassend L. monocytogenes Sv1/2a, L. monocytogenes Sv4b F2365, L. monocytogenes Sv4b H7858, 178 contigs, L. monocytogenes Sv1/2a F6854, 133 contigs, L. monocytogenes Sv4b, L. monocytogenes Sv4a, L. innocua Sv6a, L. welshimeri Sv6b, L. seeligeri Sv1/2b und/oder L. ivanovii Sv5 ausgelöst werden oder auf die genannten bevorzugten Listerien im Wesentlichen zurückgehen.

Die bevorzugten neurodegenerativen Erkrankungen sind ausgewählt aus der Gruppe umfassend Morbus Alzheimer, Morbus Parkinson, Morbus Huntington und/oder amyotrophische Lateralsklerose (ALS). Neurodegenerative Erkrankungen im Sinne der Erfindung sind ausserdem Alexander-Krankheit, AlpersHuttenlocher-Syndrom, Chorea Huntington, Creutzfeldt-Jakob-Krankheit, extrapyramidales Syndrom, Friedreich-Ataxie, kortikobasale Degeneration, Morbus Krabbe, Leukodystrophie, Lewy-KörperchenDemenz, Maskengesicht, Morbus Pick, Multiple Sklerose, Multisystematrophie, neurodegenerative Eisenablagerungen im Gehirn, progressive supranukleäre Blickparese, Shy-Drager-Syndrom, spinozerebelläre Ataxie, Synuleinopathie und/oder tropische spastische Paraparese.

Die bevorzugten Tumorerkrankungen sind ausgewählt aus der Gruppe umfassend Tumorerkrankungen des Hals-Nasen-Ohren-Bereichs, der Lunge, des Mediastinums, des Gastrointestinaltraktes, des Urogenital-Systems, des gynäkologischen Systems, der Brust, des endokrinen Systems, der Haut, Knochenund Weichtelsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppressionbezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs; das Melanom; das Hepatom; das Neuroblastom; das Papillom; das Apudo;, das Choristom; das Branchiom; das maligne Karzinoid-Syndrom; die Karzinoid-Herzerkrankung; das Karzinom (zum Beispiel Walker-Karzinom, Basalzellen-Karzinom, basosquamöses Karzinom, BrownPearce-Karzinom, duktales Karzinom, Ehrlich-Tumor, in-situ-Karzinom, Krebs-2-Karzinom, MerkelZellen-Karzinom, Schleimkrebs, nicht-kleinzelliges Bronchialkarzinom, Haferzellen-Karzinom, papilläres Karzinom, szirrhöses Karzinom, bronchiolo-alveoläres Karzinom, Bronchial-Karzinom, Plattenepithel-karzinom und Transitionalzell-Karzinom); histiocytische Funktionsstörung; Leukämie (zum Beispiel in Zusammenhang mit B-Zellen-Leukämie, Gemischt-Zellen-Leukämie, Nullzellen-Leukämie, T-ZellenLeukämie, chronische T-Zellen-Leukämie, HTLV-I I-assoziierte Leukämie, akut lymphozytische Leukä- mie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor; Reticuloendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom; Chordom; Cranio-pharyngiom; Dysgerminom; Hamartom; Mesenchymom; Mesonephrom; Myosarkom; Ameloblastom; Cementom; Odontom; Teratom; Thymom; Chorio-blastom; Adenokarzinom; Adenom; Cholangiom; Cholesteatom; Cylindrom; Cystadenocarcinom; Cystadenom; Granulosa-zelltumor; Gynadroblastom; Hidradenom; Inselzelltumor; Leydig-Zelltumor; Papillom; Sertoli-Zell-Tumor; Thekazell-tumor; Leiomyom; Leiomyosarkom; Myoblastom; Myom; Myosarkom; Rhabdomyom; Rhabdomyosarkom; Ependynom; Ganglioneurom; Gliom; Medulloblastom; Meningiom; Neurilemmom; Neuroblastom; Neuroepitheliom; Neurofibrom; Neurom; Paraganglion!; nicht-chromaifines Paragangliom; Angiokeratom; angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangio-myom, Lymphangiosarkom; Pinealom; Cystosarkom phyllodes; Hemangiosarkom; Lymphangiosarkom; Myxosarkom; Ovarialkarzinom; Sarkom (zum Beispiel Ewing-Sarkom, experi-mentell, Kaposi-Sarkom und Mastzell-Sarkom); Neoplasmen (zum Beispiel Knochen-Neoplasmen, Brust-Neoplasmen, Neoplasmendes Verdauungssystems, colorektale Neoplasmen, Leber-Neoplasmen, Pankreas-Neoplasmen, Himanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des Urogenitaltrakts); Neuro-fibromatose und zervikale Plattenepitheldysplasie. In einer weiter bevorzugten Ausführungsform ist die Krebserkrankung oder der Tumor ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchiaikarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolonund Rektumkarzinome und Analkarzinome, Urogenitaltumoren umfassend Tumoren der Nieren, der Hamleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtümoren und Karzinoidsyndrom, multiple endokrine Neoplasien, Knochenund Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehimmetastasen, Lungen-metastasen, Lebermetastasen, Knochenmetastasen, pleurale und perikardiale Metastasen und maligne Aszites.

In einer weiter bevorzugten Ausführungsform ist die Krebserkrankung oder der Tumor ausgewählt aus der Gruppe umfassend Krebserkrankungen oder Tumorerkrankungen der Mammakarzinome, der Gastrointestinaltumore, einschliesslich Kolonkarzinome, Magenkarzinome, Pankreaskarziome, Dickdarmkrebs, Dünndarm-krebs, der Ovarialkarzinome, derZervikalkarzinome, Lungen-krebs, Prostatakrebs, Nierenzellkarzinome und/oder Lebermetastasen.

Virale Erkrankungen im Sinne der Erfindung sind ausgewählt aus der Gruppe umfassend Influenza, Schnupfen, Husten, Masern, Mumps, Röteln, Ringelröteln, Drei-Tage-Fieber, Windpocken, Pfeiffersches Drüsenfieber, SARS, Zytomegalie, Durchfall, Hepatitis, Kinderlähmung, Herpes labialis, Warzen, Tollwut, Lassa-Fieber, Ebola, Marburg-Fieber, Hanta-Virus-Fieber, FSME, RSSE, Louping-illEnzephalitis, Powassan-Enzephalitis, Kyasanur-forest-Fieber, Omsk-hämorrhagisches-Fieber, Colorado-tick-Fieber, Gelbfieber, Dengue-Fieber, Japanische Enzephalitis, West-Nil-Fieber, Chikungunya-Fieber, O'nyong-nyong-Fieber, Rift-Tal-Fieber, Sandmücken-Fieber, Ross-River-Fieber, Sindbis-Fieber, Mayaro-Fieber, Murray-Valley-Enzephalitis, St. Louis-Enzephalitis, Rocio-Enzephalitis, CaliforniaEnzephalitis, Bunyamwera-Fieber, Oropouche-Fieber, AIDS, Herpes genitalis und/oder Herpes Simplex, besonders bevorzugt sind die viralen Hepatitiserkrankungen ausgewählt aus der Gruppe umfassend Hepatitis A, Hepatitis B, Hepatitis C, Hepatitis D, Hepatitis E, Hepatitis F, Hepatitis G und/oder autoimmune Hepatitis und/oder asymptomatische oder symptomatische HIV-Infektionen.

Die HIV-Infektionen können zu der Krankheit AIDS führen. Im Sinne der Erfindung ist AIDS charakterisiert bzw. klassifiziert durch ein klinisches Bild, ausgewählt aus der Gruppe umfassend
(a) asymptomatische oder symptomatische HIV-Infektionen, (b) bazilläre Angiomatosen, Entzündungen des kleinen Beckens, besonders bei Komplikationen eines Tubenoder Ovarialabszesses, ausgedehnter oder rezidivierender Herpes zoster, thrombozytopene Purpura, lang anhaltendes Fieber oder Diarrhoen, die länger als einen Monat anhalten, Listeriose, orale Harrleukoplakie, oropharyngeale Candidiosen, chronische oder schwer zu therapierende vaginale Candidosen, zervikale Dysplasien, Carcinoma in situ, periphere Neuropathie und/oder (c) Candidosen der Atemwege oder der Speiseröhre, Cytomegalievirus-Infektionen, CMV-Retinitis, HIV-bedingte Enzephalopathie, Herpes Simplex mit chronischen Ulzera (>1 Monat) oder durch Herpes simplex bedingte Bronchitis, Pneumonie oder Ösophagitis, chronische Histoplasmose, itestinale Isosporiasis, Kaposi-Sarkom, disseminierte oder extrapulmonale Kokzidiomykose, extrapulmonale Kryptokokkose, chronisch intestinale Kryptosporidiose, immunoblastisches, primär zerebrales oder Burkitt Lymphom, extrapulmonale Mykobakterien, Pneumocystis-Pneumonie, rezidivierende bakterielle Pneumonie, progressive multifokale Leukenzephalopathie, rezidivierende Salmonellen-Septikämie, Tuberkulose, zerebrale Toxoplasmose, Wasting-Symdrom und/oder invasives Zervixkarzinom. Behandlung im Sinne der Erfindung heisst sowohl Prophylaxe, Therapie, Verlaufskontrolle und/oder Nachbehandlung von Erkrankungen.

Bei der Behandlung der genannten Krankheiten ist es besonders bevorzugt, das erfindungsgemäße Arzneimittel als Gel, Puder, Pulver, Tablette, Retardtablette, Premix, Emulsion, Aufgussformulierung, Tropfen, Konzentrat, Granulat, Sirup, Pellet, Boli, Kapsel, Aerosol, Spray und/oder Inhalat zuzubereiten und/oder anzuwenden.

Bevorzugt liegt das erfindungsgemäße Arzneimittel in einer Konzentration von 0,1 bis 99,5, bevorzugt von 0,5 bis 95,0, besonders bevorzugt von 20,0 bis 80,0 Gew.-% in einer Zubereitung vor.

Bevorzugt wird diese Zubereitung oral, subkutan, intravenös, intramuskulär, intraperitoneal und/oder topisch eingesetzt.

Bevorzugt wird das erfindungsgemäße Arzneimittel in Gesamtmengen von 0,05 bis 500 mg pro kg, bevorzugt von 5 bis 100 mg pro kg Körpergewicht, je 24 Stunden eingesetzt.

Bevorzugt erfolgt das In-Kontakt-Bringen oral, über Injektion, topisch, vaginal, rektal und/oder nasal.

Ein weiterer Gegenstand der vorliegenden Erfindung ist die Verwendung wenigstens einer substituierten Verbindung der allgemeinen Formel (I) zur Herstellung einer Substanzbibliothek α-Helix induzierter Proteine und/oder Peptide.

Eine Substanzbibliothek von Proteinen und/oder Peptiden, welche eine α-Helix-Konformation aufweisen, welche durch wenigstens eine substituierte Verbindung der allgemeinen Formel (I) induziert worden ist, ist daher ebenfalls ein Gegenstand der vorliegenden Erfindung.

Ein weiterer Gegenstand der vorliegenden Erfindung ist ein Verfahren zur Herstellung einer Peptid-Bibliothek umfassend den Schritt:

Einbau einer erfindungsgemäßen Verbindung der allgemeinen Formel (I) in eine Peptidsequenz.

Bevorzugt ist ein Verfahren, in dem eine erfindungsgemäße Verbindung der allgemeinen Formel (I) auf beiden Seiten innerhalb der Sequenz jeweils unmittelbar von einem Glycin-Rest flankiert wird.

Bevorzugt ist ein ebenfalls ein Verfahren, in dem der N-Terminus der Sequenz acyliert wird.

Die zur Herstellung von Peptid-Bibliotheken üblichen Verfahren bzw. die zum Einbau von einzelnen Verbindungen in Peptidsequenzen üblichen Verfahren sind dem Fachmann bekannt. Ein bevorzugtes Verfahren zur Herstellung von Peptid-Bibliotheken ist die Festphasensynthese. Dazu erfolgt vorzugsweise ein sequentieller Aufbau der Peptide unter Einsatz geeignet geschützter Bausteine, welche kommerziell erhältlich sind. Um eine Kompatibilität der erfindungsgemäßen Verbindungen mit der jeweiligen Chemie der gewählten Festphasensynthese zu gewährleisten, sind die erfindungsgemäßen Verbindungen dazu vorzugsweise mit kompatiblen Schutzgruppen geschützt. Basiert die Festphasensynthese beispielsweise auf Fmoc-geschützten Aminosäure-Bausteinen, so werden bevorzugt auch die erfindungsgemäßen Verbindungen Fmoc-geschützt.

Eine besonders bevorzugte erfindungsgemäße Verbindung hat daher folgende Struktur: 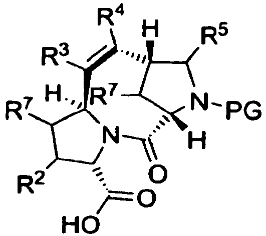 wobei vorzugsweise R² bis R⁵ und R⁷ jeweils -H sind und PG eine geeignete Schutzgruppe ist, vorzugsweise Fmoc.

Ein weiterer Gegenstand der vorliegenden Erfindung ist eine Peptid-Bibliothek, die nach dem vorstehenden Verfahren erhältlich ist.

Ein weiterer Gegenstand der Erfindung sind Verfahren zur Herstellung der erfindungsgemäßen substituierten Verbindungen der allgemeinen Formel (I).

Die in den nachstehend beschriebenen Umsetzungen zum Einsatz kommenden Chemikalien und Reaktionskomponenten sind käuflich erhältlich oder können jeweils nach üblichen, dem Fachmann bekannten Methoden hergestellt werden.

### Allgemeines Herstellungsverfahren

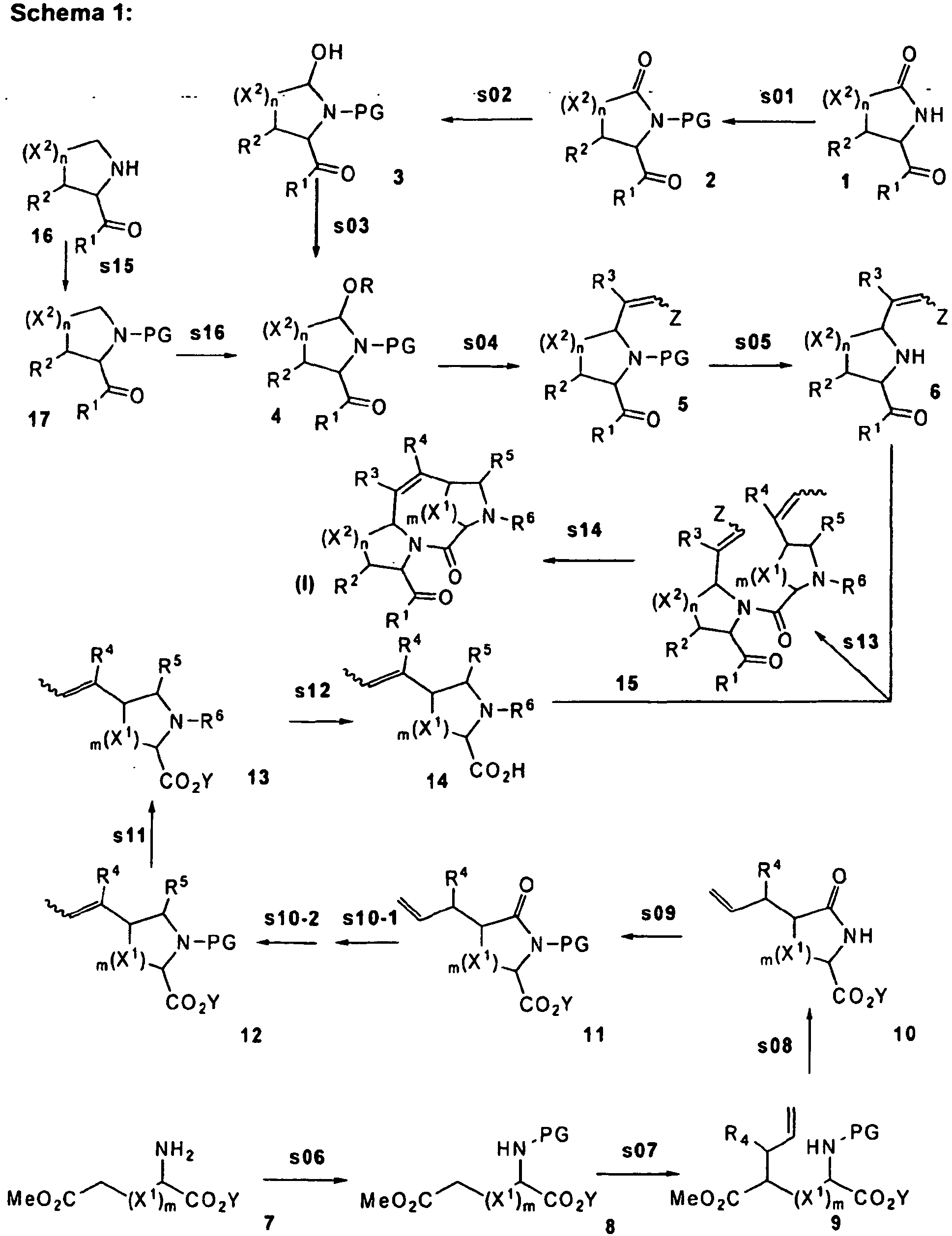

In den Schritten **s01** und **s15** wird die sekundäre Aminofunktion der Verbindungen der allgemeinen Formel **1** bzw. **16** mittels dem Fachmann geläufigen Methoden durch Einführung einer Schutzgruppe (PG, "protecting group") wie bspw. einer Boc- oder einer Moc-Schutzgruppe, ggf. in Gegenwart einer Base wie Triethylamin oder 4-Dimetyhlaminopyridin geschützt.

In Schritt **s02** wird die Keto-Gruppe der Verbindungen der allgemeinen Formel **2** mittels dem Fachmann geläufigen Methoden in einem Reaktionsmedium, vorzugsweise ausgewählt aus der Gruppe bestehend aus Diethylether, Ethanol, Essigsäure, Methanol, und Tetrahydrofuran in Gegenwart eines geeigneten Reduktionsmittels, vorzugsweise einem Metallhydrid, besonders bevorzugt ausgewählt aus der Gruppe bestehend aus Diisobutylaluminiumhydrid (DIBAL), Natriumborhydrid, Natriumcyanoborhydrid, Lithiumaluminiumhydrid oder Wasserstoff, ggf. unter Zusatz eines Katalysators, vorzugsweise ausgewählt aus der Gruppe bestehend aus Palladium, Platin, Platinoxid oder Raney-Nickel, ggf. unter Zusatz einer organischen Base ausgewählt aus der Gruppe bestehend aus Ammoniak, Triethylamin und Diisopropylethylamin zu Alkoholen der allgemeinen Formel **3** reduziert.

Im Schritt **s03** wird die OH-Funktion der Verbindungen der allgemeinen Formel **3** mittels dem Fachmann geläufigen Methoden alkyliert (bzw. gegen eine Alkoxygruppe R substituiert), bspw. durch Umsetzung mit einem Alkohol ROH in Gegenwart einer Säure wie z.B. Pyridin-p-Toluolsulfonat und so zu einer Verbindung der allgemeinen Formel 4 umgesetzt. Bevorzugte Alkohole ROH sind z.B. Methanol, Ethanol oder andere niedere Alkanole.

Alternativ können Verbindungen der allgemeinen Formel **4** auch mittels dem Fachmann geläufigen Methoden mittels einer zweistufigen Synthese ausgehend von Verbindungen der allgemeinen Formel **16** dargestellt werden, in denen zunächst die Aminofunktion in einem Schritt **s15** analog **s01** geschützt wird. Im Anschluss werden die Verbindungen der allgemeinen Formel 17 in einem Schritt **s16** elektrochemisch ggf. in Gegenwart einer Base, mit einem Alkohol ROH zu Verbindungen der allgemeinen Formel **4** umgesetzt.

Im Schritt **s04** werden Verbindungen der allgemeinen Formel **4** mittels dem Fachmann geläufigen Methoden zu Verbindungen der allgemeinen Formel **5** umgesetzt. Dabei wird bspw. ein Grignard-Reagenz Mg(CR³=CH-Z)Hal (mit Z = H oder Alkyl und Hal = Chlor, Brom oder Iod, vorzugsweise Brom), in Gegenwart eines Übergangsmetallions, vorzugsweise einer Kupfer(I)-Verbindung, ggf. in Gegenwart einer Base eingesetzt.

Im Schritt **s05** wird die zuvor eingeführte Schutzgruppe der sekundären Aminofunktion der Verbindungen der allgemeinen Formel 5 mittels dem Fachmann geläufigen Methoden wie in Gegenwart von Trialkylsilylhalogeniden oder ggf. in Gegenwart einer Säure abgespalten und so Verbindungen der allgemeinen Formel **6** erhalten.

Im Schritt **s06** wird die primäre Aminofunktion der Verbindungen der allgemeinen Formel 7 mit Y = Alkyl mittels dem Fachmann geläufigen Methoden analog zu den Schritten **s01** und **s15** mit einer geeigneten Aminschutzgruppe geschützt.

Im Schritt **s07** werden die Verbindungen der allgemeinen Formel **8** mittels dem Fachmann geläufigen Methoden am Kohlenstoffatom in α-Stellung zur COOMe-Gruppe zunächst selektiv in Gegenwart einer geeigneten Base wie z.B. Lithiumhexamethyldisilazid deprotoniert und dann mit einem Allylhalogenid Hal-CHR⁴-CH=CH₂, vorzugsweise einem Allylbromid, zu einer Verbindung der allgemeinen Formel **9** allyliert.

Im Schritt s08 werden die Verbindungen der allgemeinen Formel **9** mittels dem Fachmann geläufigen Methoden in Gegenwart einer Säure wie z.B. wässriger Phosphorsäure unter Abspaltung der Aminschutzgruppe unter Ringschluss zu Verbindungen der allgemeinen Formel **10** umgesetzt.

Im Schritt **s09** wird die sekundäre Aminofunktion der cyclischen Verbindungen der allgemeinen Formel **10** mittels dem Fachmann geläufigen Methoden analog zu den Schritten **s01, s06** und **s15** mit einer geeigneten Aminschutzgruppe geschützt. Eine besonders bevorzugte Aminschutzgruppe ist eine Boc-Gruppe.

Im Schritt **s10-1** wird die Keto-Gruppe der Verbindungen der allgemeinen Formel **11** mittels dem Fachmann geläufigen Methoden in Gegenwart eines geeigneten Reduktionsmittels, vorzugsweise einem Metallhydrid, besonders bevorzugt ausgewählt aus der Gruppe bestehend aus Diisobutyl-aluminiumhydrid (DIBAL), Natriumborhydrid, Natriumcyanoborhydrid, Lithiumaluminiumhydrid und Lithium-triethylborhydrid zum entsprechenden Alkohol reduziert und dieser im Anschluss in Gegenwart eines geeigneten Reduktionsmittels zur entsprechenden Alkyl-Verbindung (R⁵ = H) hydriert. Alternativ kann die nach der Reduktion der Keto-Gruppe entstandene Alkoholfunktion auch in eine bessere Abgangsgruppe überführt werden, bspw. durch Protonierung oder durch eine Überführung nach dem Fachmann bekannten Methoden in eine bessere Abgangsgruppe wie z.B. eine Triflat-Gruppe und dann in einer Substitutionsreaktion, bspw. durch eine metallkatalysierte Kupplungsreaktion mit R⁵-Hal mit Hal = Cl, Br, I, vorzugsweise I, durch R⁵ (R⁵ ≠ H) substituiert werden.

In Schritt **s10-2** wird die Doppelbindung in der Allylseitenkette isomerisiert, wozu bevorzugt dem Fachmann bekannte metallbasierte Katalysatoren, insbesondere auf Basis von Ru, Rh und Pd herangezogen werden.

In Schritt **s11** wird die Aminschutzgruppe von **12** analog zu **s05** abgespalten und die resultierende freie Aminofunktion mittels dem Fachmann bekannten Methoden in N-Stellung substituiert, bspw. unter Einsatz eines Halogenids R⁶-Hal, ggf. in Gegenwart einer Base, wobei Hal vorzugsweise Cl, Br oder I ist oder durch Verwendung einer Boronsäure B(OH)₂R⁶ oder eines entsprechenden Boronsäuresters, ggf. in Gegenwart eines Kupplungsreagenzes und/oder einer Base und so die Verbindung **13** gewonnen. Alternativ kann die Schutzgruppe PG aber auch nicht abgespalten werden, wenn sie selbst bereits einen erwünschten Rest R⁶ darstellt.

Im Schritt **s12** werden die Ester der allgemeinen Formel **13** mit der Gruppe CO₂Y mittels dem Fachmann geläufigen Methoden in einem Reaktionsmedium, vorzugsweise ausgewählt aus der Gruppe bestehend aus Ethanol, Methanol, MeCN, THF oder Wasser oder beliebigen Mischungen daraus, ggf. unter Zusatz einer anorganischen Base, vorzugsweise Kaliumhydroxid, Natriumhydroxid oder Lithiumhydroxid bei Temperatur von 0 °C bis 120 °C zu Säuren der allgemeinen Formel **14** gespalten.

Im Schritt **s13** wird das Amin **6** in das Amid 15 überführt. Dies kann bspw. zunächst durch Überführung der Säure **14** in ein Säurehalogenid, vorzugsweise eines Säurechlorids mittels dem Fachmann geläufigen Methoden wie bspw. durch Erhitzen von **14** in Gegenwart eines geeigneten Chlorierungsmittels beispielsweise ausgewählt aus der Gruppe (COCl)₂, PCl₃, POCl₃ oder SOCl₂ in einem Reaktionsmedium, vorzugsweise ausgewählt aus der Gruppe bestehend aus Toluol, DMF, DCM, oder Pyridin bei Temperaturen von -70°C bis 100°C, ggf. in Gegenwart von Mikrowellenstrahlung und sich anschließender Reaktion des Säurechlorids mit **6,** in einem Reaktionsmedium, vorzugsweise ausgewählt aus der Gruppe bestehend aus Ether, THF, MeCN, MeOH, EtOH, DMF und DCM, mit oder ohne Zusatz wenigstens einer organischen oder anorganischen Base, beispielsweise NEt₃, DMAP, Pyridin oder DIPEA, ggf. in Gegenwart wenigstens einer organischen Base, vorzugsweise ausgewählt aus der Gruppe bestehend aus NEt₃, DMAP, Pyridin und DIPEA, oder einer anorganischen Base bei Temperaturen von vorzugsweise -70°C bis 100°C, ggf. in Gegenwart von Mikrowellenstrahlung, erreicht werden, oder durch Reaktion von **6** mit der Säure **14,** in einem Reaktionsmedium, vorzugsweise ausgewählt aus der Gruppe bestehend aus Diethylether, Tetrahydrofuran, Acetonitril, Methanol, Ethanol, Dimethylformamid und Dichlormethan, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), Diisopropylcarbodiimied (DIC), N'-(3-Dimethylaminopropyl)-N-ethylcarbodiimid (EDCI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Pyridin, Dimethylamino-pyridin und Diisopropylethylamin vorzugsweise bei Temperaturen von -70°C bis 150°C, erreicht werden.

Im Schritt **s14** wird die Verbindung der allgemeinen Formel **15** im Zuge einer Ringschlussmetathese mittels dem Fachmann bekannten Methoden wie bspw. unter Verwendung von Grubbs- oder Schrock-Katalysatoren, vorzugsweise von Grubbs-I-oder Grubbs-II-Katalysatoren zur Verbindung der allgemeinen Formel **(I)** umgesetzt.

Alternativ zu Schritt **s14** wird die Verbindung der allgemeinen Formel **15** in Schritt **s17** zum Dialdehyd **16** umgesetzt und anschließend im Schritt **s18** einer intramolekularen McMurry-Kupplung unterzogen (siehe Schema 2). Die Synthese des Dialdehyds erfolgt vorzugsweise durch Ozonolyse und anschließender reduktiver Aufarbeitung mit beispielsweise Zn/Essigsäure, Pt/H₂, Triphenylphosphin oder Dimethylsulfid. Die McMurry-Kupplung wird mittels dem Fachmann bekannten Methoden durchgeführt. Vorzugsweise wird die McMurry-Kupplung mit niedervalentem Titan durchgeführt, welches in situ durch Reduktion von Titan(III)-chlorid oder Titan(IV)-chlorid mit z. B. Lithiumaluminiumhydrid, Zink, Mg oder Kupfer/Zink hergestellt wird.

### Zweistufige Alternative zu Schritt s14 des allgemeines Herstellungsverfahrens

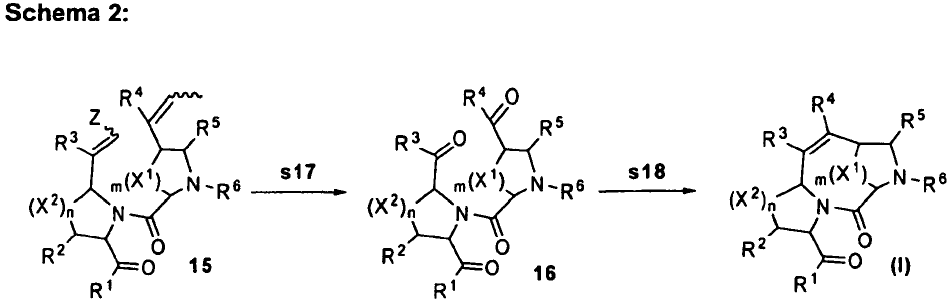

Die dem Fachmann geläufigen Methoden zur Durchführung der Reaktionsschritte **s01** bis **s18** sind den Standardwerken der Organischen Chemie zu entnehmen wie bspw. J. March, Advanced Organic Chemistry, Wiley & Sons, 6th edition, 2007; F. A. Carey, R. J. Sundberg, Advanced Organic Chemistry, Parts A and B, Springer, 5th edition, 2007); Autorenkollektiv, Compendium of Organic Synthetic Methods, Wiley & Sons. Darüber hinaus können weitere Methoden sowie Literaturverweise von den gängigen Datenbanken wie bspw. der Reaxys® Datenbank der Firma Elsevier, Amsterdam, NL oder der SciFinder® Datenbank der American Chemical Society, Washington, US, ausgegeben werden.

Die vorstehend beschriebenen Umsetzungen können jeweils unter den üblichen dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden. Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie. Sämtliche der vorstehend beschriebenen Verfahrensschritte sowie jeweils auch die Reinigung und/oder Isolierung von Zwischen- oder Endprodukten können teilweise oder vollständig unter einer Inertgasatmosphäre, vorzugsweise unter Schutzgasatmosphäre, durchgeführt werden.

Sofern die erfindungsgemäßen substituierten Verbindungen der vorstehend genannten allgemeinen Formel **(I)** bzw. die Verbindungen **1-15** nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden

### Beispiel:

Die Synthese der Zielstruktur **(I")** erfolgte durch Kupplung der Vinylprolinderivate **6a** und **14a** und einer nachfolgenden Ringschlussmetathese zum Aufbau des charakteristischen Ringsystems.

Die Synthese des Vinylprolinderivats **6a** wurde ausgehend von L-Pyroglutaminsäure **(1a)** in sechs Schritten durchgeführt (Schema 3). Als Alternative wurde die Synthese von **6a** fünfstufig ausgehend von L-Prolin **(16a)** mit Hilfe einer elektrochemischen Oxidation von **17a** als Schlüsselschritt (Schritt **p)** entwickelt. Als ein weiteres geeignetes Intermediat wurde die Verbindung **6b** durch Cu-katalysierte Substititution der Methoxygruppe in **4a** synthetisiert (Schema 3).

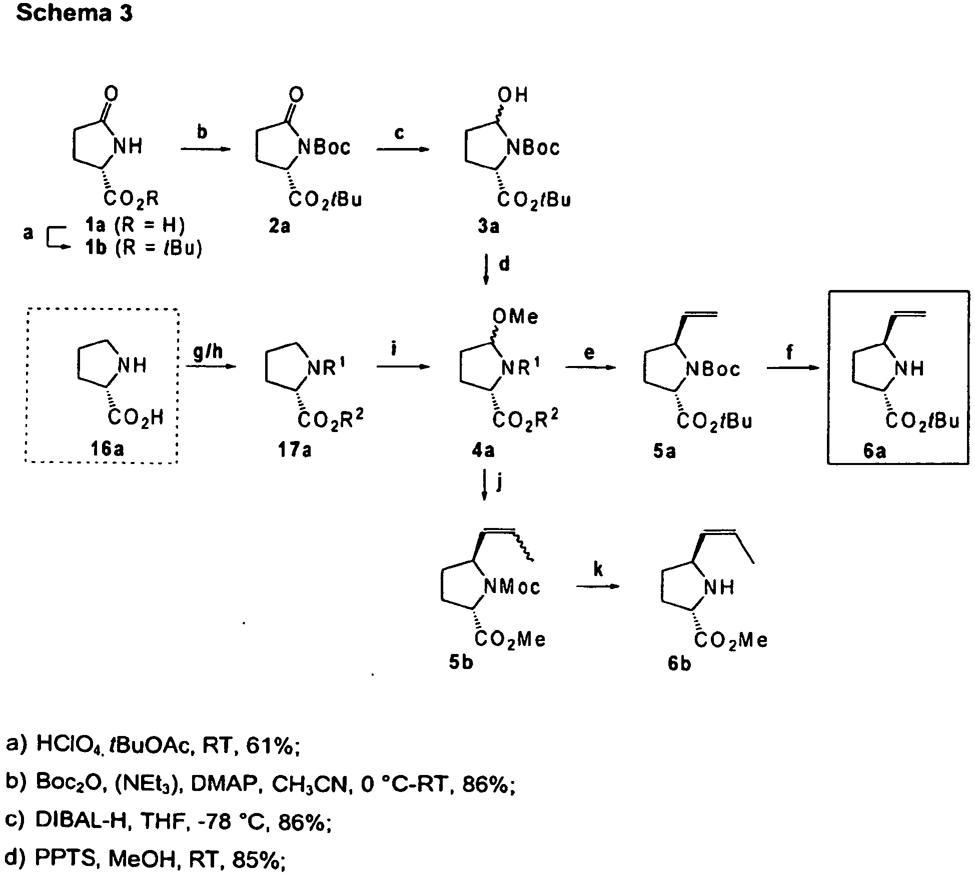

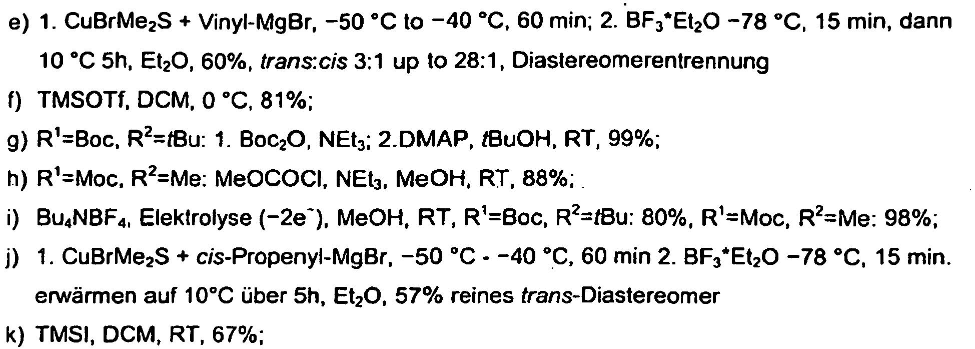

Ausgehend von L-Glutaminsäuremethylesterhydrochlorid **(7a)** wurde das Vinylprolinderivat **14a** in sieben Schritten hergestellt (Schema 4). Schlüsselschritte waren zum einen die diastereoselektive Allylierung von **8a** zu **9a** und die Isomerisierung der Doppelbindung **(12a** nach **12b).**

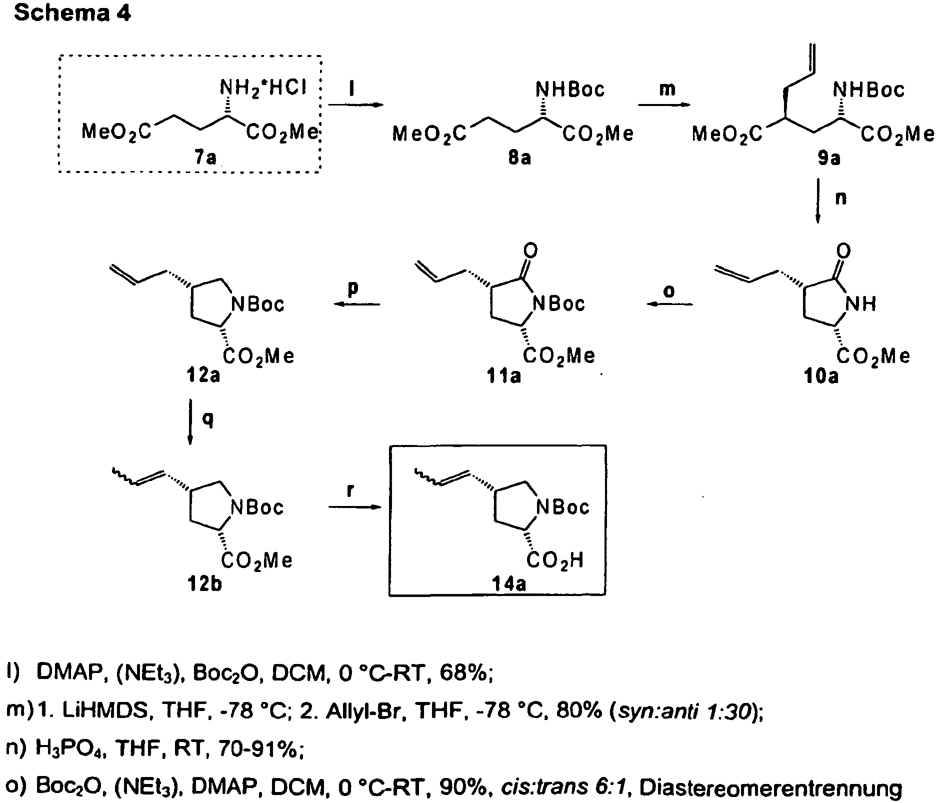

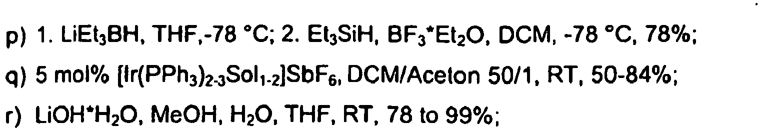

Die Kupplung der Vinylprolinderivate **6a** und **14a** zum Dipeptid **15a** gelang mit schwankenden Ausbeuten (20-76%). Die anschließende Metathese zu **(I')**, d.h. der erwartungsgemäß schwierige Ringschluss unter Bildung des 8-gliedrigen Ringes, konnte mit befriedigender Ausbeute (57%) realisiert werden, zumindest wenn relativ hohe Ladungen (30 mol%) des Grubbs II-Katalysators eingesetzt wurden. Anhaftende Ru-Rückstände und die damit verbundene Instabilität des Metatheseproduktes **(I')** bedingten allerdings, dass das eigentliche Zielprodukt (1") bislang nur in verunreinigter Form (und schlechter Ausbeute) erhalten wurde (Schema 5).

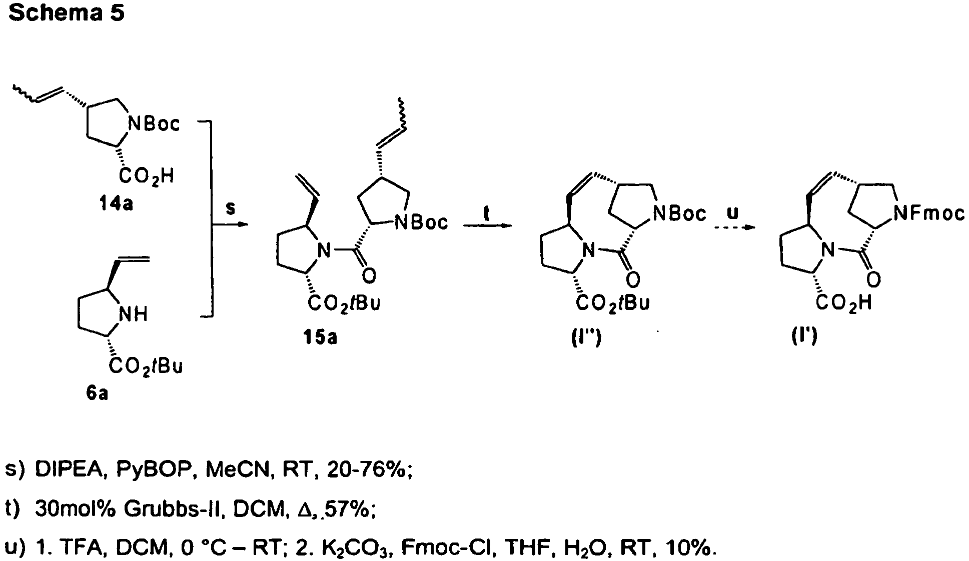

## Patentansprüche

1. Substituierte Verbindungen der allgemeinen Formel (I) 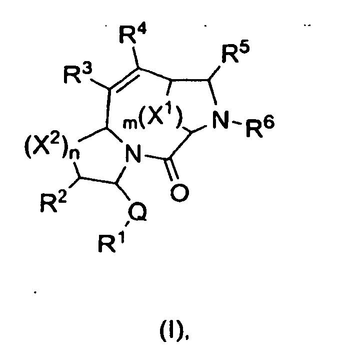 worin
Q für C(=O) oder S(=O)₂ steht;
X¹ und X² jeweils unabhängig voneinander für O, S, CH₂ oder CHR⁷ stehen, wobei R⁷ für C₁₋₁₀-Alkyl, gesättigt oder ungesättigt, verzweigt oder unverzweigt, unsubstituiert oder einfach oder mehrfach substituiert, steht;
m für 1, 2 oder 3 steht;
n für 0, 1, 2 oder 3 steht;
R¹ für C₁₋₁₀-Alkyl, gesättigt oder ungesättigt, verzweigt oder unverzweigt, unsubstituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl-verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; OR⁸; NR⁸R⁹; NHC(=O)R⁸; NR⁸R⁹; oder NH-S(=O)₂R⁸; steht,
wobei R⁸ und R⁹ jeweils unabhängig voneinander für H; C₁₋₁₀-Alkyl, gesättigt oder ungesättigt, verzweigt oder unverzweigt, unsubstituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl-verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; stehen;
oder R¹ für -NHCHR^{a}C(=O)-R^{b} steht, wobei
R^{a} ausgewählt ist aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NHC(=NH)NH₂, -CH₂-Imidazolyl, -CH₂CO₂H, und -CH₂CH₂CO₂H; und R^{b}
ausgewählt ist aus -OH und -Peptidyl, wobei das Peptidyl, über seinen N-Terminus kovalent gebunden ist;
R² und R⁵ jeweils unabhängig voneinander für H; C₁₋₁₀-Alkyl, gesättigt oder ungesättigt, verzweigt oder unverzweigt, unsubstituiert oder einfach oder mehrfach substituiert, stehen;
R³ und R⁴ jeweils unabhängig voneinander für H; C₁₋₁₀-Alkyl, gesättigt oder ungesättigt, verzweigt oder unverzweigt, unsubstituiert oder einfach oder mehrfach substituiert; Aryl oder Heteroaryl, jeweils unsubstituiert oder einfach oder mehrfach substituiert; stehen;
R⁶ für H; C₁₋₁₀-Alkyl, gesättigt oder ungesättigt, verzweigt oder unverzweigt, unsubstituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über C₁₋₈-Alkyl-verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; C(=O)R¹⁰; C(=O)OR¹⁰; C(=O)NR¹⁰R¹¹; S(=O)₂-R¹⁰; S(=O)₂OR¹⁰; oder S(=O)₂NR¹⁰R¹¹; steht,
wobei R¹⁰ und R¹¹ jeweils unabhängig voneinander für H; C₁₋₁₀-Alkyl, gesättigt oder ungesättigt, verzweigt oder unverzweigt, unsubstituiert oder einfach oder mehrfach substituiert; C₃₋₁₀-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert, stehen; über C₁₋₈-Alkyl verbrücktes C₃₋₁₀-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C₁₋₈-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; stehen;
oder R⁶ für -C(=O)-CHR^{y}NHR^{z} steht, wobei
R^{y} ausgewählt ist aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NHC(=NH)NH₂, -CH₂-Imidazolyl, -CH₂CO₂H, und -CH₂CH₂CO₂H; und
R^{z} ausgewählt ist aus -H und -C(=O)-C₁₋₈-Alkyl;
worin "Alkyl substituiert", "Heterocyclyl substituiert" und "Cycloalkyl substituiert" an den entsprechenden Resten für die Substitution eines oder mehrerer Wasserstoffatome jeweils unabhängig voneinander durch F; Cl; Br; I; NO₂; CN; CF₃; C₁₋₈-Alkyl; Phenyl ; C(=O)OH; C(=O)O-C₁₋₈-Alkyl; C(=O)O-Phenyl; C(=O)NH₂; C(=O)NH-C₁₋₈-Alkyl; C(=O)NH-Phenyl; C(=O)N(C₁₋₈-Alkyl)₂; OH; =O; OCF₃; O-C₁₋₈-Alkyl; O-C(=O)-C₁₋₈-Alkyl; SH; SCF₃; S-C₁₋₈-Alkyl; S(=O)₂OH; S(=O)₂C₁₋₈-Alkyl; S(=O)₂O-C₁₋₈-Alkyl; S(=O)₂NH-C₁₋₈-Alkyl; S(=O)₂N(C₁₋₈-Alkyl)₂; NH₂; NH-C₁₋₈-Alkyl; NH-Phenyl; N(C₁₋₈-Alkyl)₂; NH-C(=O)-C₁₋₈-Alkyl; NH-S(=O)₂-C₁₋₈-Alkyl, steht;
worin "Aryl substituiert" und "Heteroaryl substituiert" an den entsprechenden Resten für die Substitution eines oder mehrerer Wasserstoffatome jeweils unabhängig voneinander durch F; Cl; Br; I; NO₂; CN; CF₃; C₁₋₈-Alkyl; Phenyl; C(=O)OH; C(=O)O-C₁₋₈-Alkyl; C(=O)O-Phenyl; C(=O)NH₂; C(=O)NH-C₁₋₈-Alkyl; C(=O)NH-Phenyl; C(=O)N(C₁₋₈-Alkyl)₂; OH; OCF₃; O-C₁₋₈-Alkyl; O-C(=O)-C₁₋₈-Alkyl; SH; SCF₃; S-C₁₋₈-Alkyl; S(=O)₂OH; S(=O)₂C₁₋₈-Alkyl; S(=O)₂O-C₁₋₈-Alkyl; S(=O)₂NH-C₁₋₈-Alkyl; S(=O)₂N(C₁₋₈-Alkyl)₂; NH₂; NH-C₁₋₈-Alkyl; NH-Phenyl; N(C₁₋₈-Alkyl)₂; NH-C(=O)-C₁₋₈-Alkyl; NH-S(=O)₂-C₁₋₈-Alkyl, steht;
jeweils in Form der freien Verbindungen; der Racemate; der Enantiomere, Diastereomere, Mischungen der Enantiomere oder Diastereomere oder eines einzelnen Enantiomers oder Diastereomers; oder in Form der Salze physiologisch verträglicher Säuren oder Basen; oder ggf. in Form von Solvaten.

2. Substituierte Verbindungen nach Anspruch 1, **dadurch gekennzeichnet, dass**
X¹ und X² jeweils für CH₂ stehen;
m für 1 oder 2, vorzugsweise für 1 steht;
n für 0, 1 oder 2, vorzugsweise für 1 steht.

3. Substituierte Verbindungen nach Anspruch 1 oder 2, **dadurch gekennzeichnet, dass**
R¹ für OR⁸; NHR⁸ oder NHC(=O)R⁸; steht,
wobei R⁸ für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, C(=O)OH, C(=O)OC₁₋₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl; Aryl, unsubstituiert oder einfach oder mehrfach substituiert mit einem oder mehreren Substituenten jeweils unabhängig voneinander ausgewählt aus der Gruppe bestehend aus F, Cl, Br, I, C₁₋₄-Alkyl, C(=O)OH, C(=O)OC₁₋₄-Alkyl, C(=O)C₁₄-Alkyl, C(=O)NH-C₁₋₄-Alkyl, OH, O-C₁₋₄-Alkyl, NH₂, NHC(=O)C₁₄-Alkyl, NHS(=O)₂C₁₋₄-Alkyl, S(=O)₂OH, S(=O)₂OC₁₋₄-Alkyl und S(=O)₂C₁₋₄-Alkyl; oder über C₁₋₈-Alkyl-verbrücktes Aryl, unsubstituiert oder einfach oder mehrfach substituiert mit einem oder mehreren Substituenten jeweils unabhängig voneinander ausgewählt aus der Gruppe bestehend aus F, Cl, Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl; wobei die Alkylkette jeweils verzweigt oder unverzweigt, gesättigt oder ungesättigt, unsubstituiert sein kann; steht;
oder R¹ für -NHCHR^{a}C(=O)-R^{b} steht, wobei
R^{a} ausgewählt ist aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NHC(=NH)NH₂, -CH₂-Imidazolyl, -CH₂CO₂H, und -CH₂CH₂CO₂H; und
R^{b} ausgewählt ist aus -OH und -Peptidyl, wobei das Peptidyl, über seinen N-Terminus kovalent gebunden ist.

4. Substituierte Verbindungen nach einem der Ansprüche 1-3, **dadurch gekennzeichnet, dass**
R² und R⁵ jeweils unabhängig voneinander für H; C₁₋₁₀-Alkyl, gesättigt oder ungesättigt, verzweigt oder unverzweigt, unsubstituiert, stehen;
R³ und R⁴ jeweils unabhängig voneinander für H; C₁₋₁₀-Alkyl, gesättigt oder ungesättigt, verzweigt oder unverzweigt, unsubstituiert; stehen.

5. Substituierte Verbindungen nach einem der Ansprüche 1-4, **dadurch gekennzeichnet, dass**
R⁶ für H; C₁₋₁₀-Alkyl, gesättigt oder ungesättigt, verzweigt oder unverzweigt, substituiert mit einem oder mehreren Substituenten jeweils unabhängig voneinander ausgewählt aus der Gruppe bestehend aus F, Cl. Br, I, C(=O)OH, C(=O)OC₁₋₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl;
oder für C(=O)R¹⁰; C(=O)OR¹⁰; C(=O)NR¹⁰R¹¹: S(=O)₂-R¹⁰; S(=O)₂OR¹⁰; oder S(=O)₂NR¹⁰R¹¹ steht,
wobei R¹⁰ und R¹¹ jeweils unabhängig voneinander für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₄-Alkyl; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl, stehen; oder über C₁₋₈-Alkyl-verbrücktes Aryl oder Heteroaryl, jeweils unsubstituiert oder einfach uder mehrfach substituiert mit einem oder mehreren Substituenten jeweils unabhängig voneinander ausgewählt aus der Gruppe bestehend aus F, Cl, Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl; wobei die Alkylkette jeweils verzweigt oder unverzweigt, gesättigt oder ungesättigt, unsubstituiert sein kann; stehen;
oder R⁶ für -C(=O)-CHR^{y}NHR^{z} steht, wobei
R^{y} ausgewählt ist aus der Gruppe bestehend aus -H, -CH₃,-CH(CH₃)₂, -CH₂CH(CH₃)₂, -CH(CH₃)CH₂CH₃, -CH₂CH₂SCH₃,-CH₂C₆H₅, -CH₂-Indolyl, -CH₂OH, -CH(OH)CH₃, -CH₂C(=O)NH₂,CH₂CH₂C(=O)NH₂, -CH₂-C₆H₄-OH, -CH₂SH,-CH₂CH₂CH₂CH₂NH₂, -CH₂CH₂CH₂NHC(=NH)NH₂, -CH₂-Imidazolyl, -CH₂CO₂H, und -CH₂CH₂CO₂H; und
R^{z} ausgewählt ist aus -H und -C(=O)-C₁₋₈-Alkyl.

6. Substituierte Verbindungen nach einem der Ansprüche 1-5, **dadurch gekennzeichnet, dass**
X¹ und X² jeweils für CH₂ stehen;
m und n jeweils für 1 stehen;
R¹ für OR⁸ steht,
wobei R⁸ für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C(=O)OH,C(=O)OC₁₋₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₄-Alkyl; oder über C₁₋₈-Alkyl-verbrücktes Aryl, unsubstituiert oder einfach oder mehrfach substituiert mit einem oder mehreren Substituenten jeweils unabhängig voneinander ausgewählt aus der Gruppe bestehend aus F, Cl, Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, S(=O)₂OH, S(=O)₂OC₁₋₄-Alkyl und S(=O)₂C₁₋₄-Alkyl; wobei die Alkylkette jeweils verzweigt oder unverzweigt, gesättigt oder ungesättigt, unsubstituiert sein kann; steht;
oder R¹ für -NHCHR^{a}C(=O)-R^{b} steht, wobei
R^{a} ausgewählt ist aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH_{2,} -CH₂CH₂CH₂NHC(=NH)NH₂, -CH₂-Imidazolyl, -CH₂CO₂H, und -CH₂CH₂CO₂H; und
R^{b} ausgewählt ist aus -OH und -Peptidyl, wobei das Peptidyl, über seinen N-Terminus kovalent gebunden ist;
R² und R⁵ jeweils für H stehen;
R³ und R⁴ jeweils für H stehen;
R⁶ für H; C₁₋₁₀-Alkyl, gesättigt oder ungesättigt, verzweigt oder unverzweigt, substituiert mit einem oder mehreren Substituenten jeweils unabhängig voneinander ausgewählt aus der Gruppe bestehend aus C(=O)OH, C(=O)OC₁₋₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄-Alkyl und NHS(=O)₂C₁₋₄-Alkyl;
oder für C(=O)R^{10;} C(=O)OR¹⁰; C(=O)NR¹⁰R¹¹; S(=O)₂-R¹⁰; S(=O)₂OR¹⁰; oder S(=O)₂NR¹⁰R¹¹ steht,
wobei R¹⁰ und R¹¹ jeweils unabhängig voneinander für H; C₁₋₁₀-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, =O, NH₂, NHC(=O)C₁₋₄-Alkyl, NHS(=O)₂C₁₋₄-Alkyl; Aryl, unsubstituiert oder einfach oder mehrfach substituiert mit einem oder mehreren Substituenten jeweils unabhängig voneinander ausgewählt aus der Gruppe bestehend aus C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, stehen; oder über C₁₋₈-Alkyl-verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, C₁₋₄-Alkyl, C(=O)OH, C(=O)OC₁₋₄-Alkyl, C(=O)C₁₋₄-Alkyl, C(=O)NH-C₁₋₄-Alkyl, OH, O-C₁₋₄-Alkyl, NH₂, NHC(=O)C₁₋₄-Alkyl, NHS(=O)₂C₁₋₄-Alkyl, wobei die Alkylkette jeweils verzweigt oder unverzweigt, gesättigt oder ungesättigt, unsubstituiert sein kann; stehen;
oder R⁶ für -C(=O)-CHR^{y}NHR^{z} steht, wobei
R^{y} ausgewählt ist aus der Gruppe bestehend aus -H, -CH₃, -CH(CH₃)₂, -CH₂CH(CH₃)₂, -CH(CH₃)CH₂CH₃, -CH₂CH₂SCH₃, -CH₂C₆H₅, -CH₂-Indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NHC(=NH)NH₂, -CH₂-Imidazolyl, -CH₂CO₂H, und -CH₂CH₂CO₂H; und
R^{z} ausgewählt ist aus -H und -C(=O)-C₁₋₈-Alkyl.

7. Verwendung wenigstens einer Verbindung der allgemeinen Formel (I) nach einem der Ansprüche 1-6 für die Induktion einer α-Helix-Konformation in wenigstens einem Protein und/oder Peptid.

8. Arzneimittel enthaltend wenigstens eine Verbindung der allgemeinen Formel (I) nach einem der Ansprüche 1-6
sowie gegebenenfalls einen oder mehrere pharmazeutisch verträgliche Hilfsstoffe und/oder gegebenenfalls weitere Wirkstoffe.

9. Verwendung wenigstens einer substituierten Verbindung der allgemeinen Formel (I) nach einem der Ansprüche 1-6 zur Herstellung eines Arzneimittels zur Behandlung und/oder Prophylaxe von einer oder mehrerer Erkrankungen und/oder Störungen, welche zumindest teilweise mit der Induktion einer α-Helix-Konformation in wenigstens einem Protein und/oder wenigstens einem Peptid assoziiert sind.

10. Verwendung wenigstens einer substituierten Verbindung der allgemeinen Formel (I) nach einem der Ansprüche 1-6 zur Herstellung eines Arzneimittels zur Behandlung und/oder Prophylaxe von einer oder mehrerer Erkrankungen und/oder Störungen, welche ausgewählt sind aus der Gruppe bestehend aus bakteriellen und viralen Infektionskrankeiten, neurodegenerativen Erkrankungen und Tumorerkrankungen.

11. Verwendung wenigstens einer substituierten Verbindung der allgemeinen Formel (I) nach einem der Ansprüche 1-6 zur Herstellung einer Substanzbibliothek α-Helix induzierter Proteine und/oder Peptide.

12. Substanzbibliothek α-Hetix induzierter Proteine und/oder Peptide enthaltend wenigstens eine substituierte Verbindung der allgemeinen Formel (I) nach einem der Ansprüche 1-6.

13. Verfahren zur Herstellung einer Peptid-Bibliothek umfassend den Schritt:
Einbringen einer Verbindung nach einem der Ansprüche 1-6 in eine Peptidsequenz.

14. Verfahren nach Anspruch 13, **dadurch gekennzeichnet, dass** die Verbindung nach einem der Ansprüche 1-6 auf beiden Seiten innerhalb der Sequenz jeweils unmittelbar von einem Glycin-Rest flankiert wird.

15. Eine Peptid-Bibliothek erhältlich nach Anspruch 13 oder 14.

## Claims

1. A substituted compound of the general formula (I) 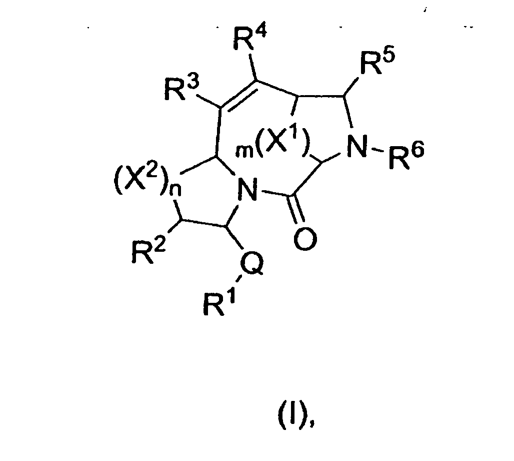 in which
Q is C(=O) or S(=O)₂;
X¹ and X² are each independently 0, S, CH₂ or CHR⁷,
where R⁷ is C₁₋₁₀-alkyl, saturated or unsaturated, branched or unbranched, unsubstituted or mono- or polysubstituted;
m is 1, 2 or 3;
n is 0, 1, 2 or 3;
R¹ is C₁₋₁₀-alkyl, saturated or unsaturated, branched or unbranched, unsubstituted or mono- or polysubstituted; C₃₋₁₀-cycloalkyl or heterocyclyl, each saturated or unsaturated, unsubstituted
or mono- or polysubstituted; aryl or heteroaryl, each unsubstituted or mono- or polysubstituted; C₁₋₈-alkyl-bridged C₃₋₁₀-cycloalkyl or heterocyclyl, each saturated or unsaturated, unsubstituted or mono- or polysubstituted, where the alkyl chain in each case may be branched or unbranched, saturated or unsaturated, unsubstituted, mono- or polysubstituted; or C₁₋₈-alkyl-bridged aryl or heteroaryl, each unsubstituted or mono- or polysubstituted, where the alkyl chain in each case may be branched or unbranched, saturated or unsaturated, unsubstituted, mono- or polysubstituted; OR⁸; NR⁸R⁹; NHC(=O)R⁸; NR⁸R⁹; or NH-S(=O)₂R⁸; where R⁸ and R⁹ are each independently H; C₁₋₁₀-alkyl, saturated or unsaturated, branched or unbranched, unsubstituted or mono- or polysubstituted; C₃₋₁₀-cycloalkyl or heterocyclyl, each saturated or unsaturated, unsubstituted or mono- or polysubstituted; aryl or heteroaryl, each unsubstituted or mono- or polysubstituted; C₁₋₈-alkyl-bridged C₃₋₁₀-cycloalkyl or heterocyclyl, each saturated or unsaturated, unsubstituted or mono- or polysubstituted, where the alkyl chain in each case may be branched or unbranched, saturated or unsaturated, unsubstituted, mono- or polysubstituted; or C₁₋₈-alkyl-bridged aryl or heteroaryl, each unsubstituted or mono- or polysubstituted, where the alkyl chain in each case may be branched or unbranched, saturated or unsaturated, unsubstituted, mono- or polysubstituted;
or R¹ is -NHCHR^{a}C(=O)-R^{b} where
R^{a} is selected from the group consistingof -H, -CH₃, -CH(CH₃)₂, -CH₂CH(CH₃)₂,-CH(CH₃)CH₂CH₃, -CH₂CH₂SCH₃, -CH₂C₆H₅,-CH₂-indolyl, -CH₂OH, -CH(OH)CH₃,-CH₂C(=O)NH₂, CH₂CH₂C(=O)NH₂, -CH₂-C₆H₄-OH,-CH₂SH, -CH₂CH₂CH₂CH₂NH₂,-CH₂CH₂CH₂NHC (=NH) NH₂, -CH₂-imidazolyl,-CH₂CO₂H, and -CH₂CH₂CO₂H; and
R^{b} is selected from -OH and -peptidyl, where the peptidyl is bonded covalently via its N terminus;
R² and R⁵ are each independently H; C₁₋₁₀-alkyl, saturated or unsaturated, branched or unbranched, unsubstituted or mono- or polysubstituted;
R³ and R⁴ are each independently H; C₁₋₁₀-alkyl, saturated or unsaturated, branched or unbranched, unsubstituted or mono- or polysubstituted; aryl or heteroaryl, each unsubstituted or mono- or polysubstituted;
R⁶ is H; C₁₋₁₀-alkyl, saturated or unsaturated, branched or unbranched, unsubstituted or mono- or polysubstituted; C₃₋₁₀-cycloalkyl or heterocyclyl, each saturated or unsaturated, unsubstituted or mono- or polysubstituted; aryl or heteroaryl, each unsubstituted or mono- or polysubstituted; C₁₋₈-alkyl-bridged C₃₋₁₀-cycloalkyl or heterocyclyl, each saturated or unsaturated, unsubstituted or mono- or polysubstituted, where the alkyl chain in each case may be branched or unbranched, saturated or unsaturated, unsubstituted, mono- or polysubstituted; or C₁₋₈-alkyl-bridged aryl or heteroaryl, each unsubstituted or mono- or polysubstituted, where the alkyl chain in each case may be branched or unbranched, saturated or unsaturated, unsubstituted, mono- or polysubstituted; C(=O)R¹⁰; C(=O)OR¹⁰; C(=O)NR¹⁰R¹¹; S(=O)₂-R¹⁰; S(=O)₂OR¹⁰; or S(=O)₂NR¹⁰R¹¹;
where R¹⁰ and R¹¹ are each independently H; C₁₋₁₀-alkyl, saturated or unsaturated, branched or unbranched, unsubstituted or mono- or polysubstituted; C₃₋₁₀-cycloalkyl or heterocyclyl, each saturated or unsaturated, unsubstituted or mono- or polysubstituted; aryl or heteroaryl, each unsubstituted or mono- or polysubstituted; C₁₋₈-alkyl-bridged C₃₋₁₀-cycloalkyl or heterocyclyl, each saturated or unsaturated, unsubstituted or mono- or polysubstituted, where the alkyl chain in each case may be branched or unbranched, saturated or unsaturated, unsubstituted, mono- or polysubstituted; or C₁₋₈-alkyl-bridged aryl or heteroaryl, each unsubstituted or mono- or polysubstituted, where the alkyl chain in each case may be branched or unbranched, saturated or unsaturated, unsubstituted, mono- or polysubstituted;
or R⁶ is -C(=O)-CHR^{y}NHR^{z} where
R^{y} is selected from the group consisting of -H, -CH₃, -CH(CH₃)₂, -CH₂CH(CH₃)₂, -CH(CH₃)CH₂CH₃, -CH₂CH₂SCH₃, -CH₂C₆H₅, -CH₂-indolyl, -CH₂OH, -CH(OH)CH₃, -CH₂C(=O)NH₂, CH₂CH₂C (=O) NH₂, -CH₂-C₆H₄-OH, -CH₂SH, -CH₂CH₂CH₂CH₂NH₂, -CH₂CH₂CH₂NHC (=NH) NH₂, -CH₂-imidazolyl, -CH₂CO₂H, and -CH₂CH₂CO₂H; and
R^{z} is selected from -H and -C(=O)-C₁₋₈-alkyl;
in which "alkyl substituted", "heterocyclyl substituted" and "cycloalkyl substituted" on the corresponding radicals represents the substitution of one or more hydrogen atoms in each case independently by F; Cl; Br; I; NO₂; CN; CF₃; C₁₋₈-alkyl; phenyl; C(=O)OH; C(=O)O-C₁₋₈-alkyl; C(=O)O-phenyl; C(=O)NH₂; C(=O)NH-C₁₋₈-alkyl; C(=O)NH-phenyl; C(=O)N(C₁₋₈-alkyl)₂; OH; =O; OCF₃; O-C₁₋₈-alkyl; O-C(=O)-C₁₋₈-alkyl; SH; SCF₃; S-C₁₋₈-alkyl; S(=O)₂OH; S(=O)₂C₁₋₈-alkyl; S(=O)₂O-C₁₋₈-alkyl; S(=O)₂NH-C₁₋₈-alkyl; S(=O)₂N(C₁₋₈-alkyl)₂; NH₂; NH-C₁₈-alkyl; NH-phenyl; N(C₁₋₈-alkyl)₂; NH-C(=O)-C₁₋₈-alkyl; NH-S(=O)₂-C₁₋₈-alkyl;
in which "aryl substituted" and "heteroaryl substituted" on the corresponding radicals represents the substitution of one or more hydrogen atoms in each case independently by F; Cl; Br; I; NO₂; CN; CF₃; C₁₋₈-alkyl; phenyl; C(=O)OH; C(=O)O-C₁₋₈-alkyl; C(=O)O-phenyl; C(=O)NH₂; C(=O)NH-C₁₋₈-alkyl; C(=O)NH-phenyl; C(=O)N(C₁₋₈-alkyl)₂; OH; OCF₃; O-C₁₋₈-alkyl; O-C(=O)-C₁₋₈-alkyl; SH; SCF₃; S-C₁₋₈-alkyl; S(=O)₂OH; S(=O)₂ C₁₋₈-alkyl; S(=O)₂O-C₁₋₈-alkyl; S(=O)₂NH-C₁₋₈-alkyl; S(=O)₂N(C₁₋₈-alkyl)₂; NH₂; NH-C₁₋₈-alkyl; NH-phenyl; N(C₁₋₈-alkyl)₂; NH-C (=O)-C₁₋₈-alkyl; NH-S(=O)₂-C₁₋₈-alkyl;
in each case in the form of the free compounds; of the racemates; of the enantiomers, diastereomers, mixtures of the enantiomers or diastereomers, or of a single enantiomer or diastereomer; or in the form of the salts of physiologically compatible acids or bases; or if appropriate in the form of solvates.

2. A substituted compound as claimed in claim 1, **characterized in that**
X¹ and X² are each CH₂;
m is 1 or 2, preferably 1;
n is 0, 1 or 2, preferably 1.

3. A substituted compound as claimed in claim 1 or 2, **characterized in that**
R¹ is OR⁸; NHR⁸ or NHC(=O)R⁸;
where R⁸ is H; C₁₋₁₀-alkyl, saturated or unsaturated, branched or unbranched, unsubstituted or mono- or polysubstituted by one or more substituents each independently selected from the group consisting of F, Cl, Br, I, C(=O)OH, C(=O)OC₁₋₄-alkyl, C(=O)C₁₋₄-alkyl, C (=O) NH-C₁₋₄-alkyl, OH, O-C₁₋₄-alkyl, =O, NH₂, NHC(=O)C₁₋₄-alkyl, NHS(=O)₂C₁₋₄-alkyl, S(=O)₂OH, S(=O)₂OC₁₋₄-alkyl and S(=O)₂C₁₋₄-alkyl; aryl, unsubstituted or mono- or polysubstituted by one or more substituents each independently selected from the group consisting of F, Cl, Br, I, C₁₋₄-alkyl, C(=O)OH, C(=O)OC₁₋₄-alkyl, C(=O)C₁₋₄-alkyl, C(=O)NH-C₁₋₄-alkyl, OH, O-C₁₋₄-alkyl, NH₂, NHC(=O)C₁₋₄-alkyl, NHS(=O)₂C₁₋₄-alkyl, S(=O)₂OH, S(=O)₂C₁₋₄-alkyl and S(=O)₂C₁₋₄-alkyl; or C₁₋₈-alkyl-bridged aryl, unsubstituted or mono- or polysubstituted by one or more substituents each independently selected from the group consisting of F, Cl, Br, I, C₁₋₄-alkyl, C(=O)OH, C(=O)OC₁₋₄-alkyl, C(=O)C₁₋₄-alkyl, C(=O)NH-C₁₋₄-alkyl, OH, O-C₁₋₄-alkyl, NH₂, NHC (=0)C₁₋₄-alkyl, NHS(=O)₂C₁₋₄-alkyl, S(=O)₂OH, S(=O)₂OC₁₋₄-alkyl and S(=O)₂C₁₋₄-alkyl; where the alkyl chain may in each case be branched or unbranched, saturated or unsaturated, unsubstituted;
or R¹ is -NHCHR^{a}C(=O)-R^{b} where
R^{a} is selected from the group consisting of -H, -CH₃, -CH(CH₃)₂, -CH₂CH(CH₃)₂, -CH(CH₃)CH₂CH₃, -CH₂CH₂SCH₃, -CH₂C₆H₅, -CH₂-indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NHC (=NH) NH₂, -CH₂-imidazolyl, -CH₂CO₂H, and -CH₂CH₂CO₂H; and
R^{b} is selected from -OH and -peptidyl, where the peptidyl is bonded covalently via its N terminus.

4. A substituted compound as claimed in any of claims 1-3, **characterized in that**
R² and R⁵ are each independently H; C₁₋₁₀-alkyl, saturated or unsaturated, branched or unbranched, unsubstituted;
R³ and R⁴ are each independently H; C₁₋₁₀-alkyl, saturated or unsaturated, branched or unbranched, unsubstituted.

5. A substituted compound as claimed in any of claims 1-4, **characterized in that**
R⁶ is H; C₁₋₁₀-alkyl, saturated or unsaturated, branched or unbranched, substituted by one or more substituents each independently selected from the group consisting of F, Cl, Br, I, C(=O)OH, C (=O)OC₁₋₄-alkyl, C (=O)C₁₋₄-alkyl, C (=O)NH-C₁₋₄-alkyl, OH, O-C₁₋₄-alkyl, =O, NH₂, NHC(=O)C₁₋₄-alkyl, NHS(=O)₂C₁₋₄-alkyl, S(=O)₂OH, S(=O)₂OC₁₋₄-alkyl and S(=O)₂C₁₋₄-alkyl;
or is C(=O)R¹⁰; C(=O)OR¹⁰; C(=O)NR¹⁰R¹¹; S(=O)₂-R¹⁰; S(=O)₂OR¹⁰; or S(=O)₂NR¹⁰R¹¹,
where R¹⁰ and R¹¹ are each independently H; C₁₋₁₀-alkyl, saturated or unsaturated, branched or unbranched, unsubstituted or mono- or polysubstituted by one or more substituents each independently selected from the group consisting of F, Cl, Br, I, C₁₋₄-alkyl C(=O)OH, C(=O)OC₁₋₄-alkyl, C (=O)C₁₋₄-alkyl, C (=O)NH-C₁₋₄-alkyl, OH, O-C₁₋₄-alkyl, =O, NH₂, NHC(=O)C₁₋₄-alkyl, NHS(=O)₂C₁₋₄-alkyl, S(=O)₂OH, S(=O)₂OC₁₋₄-alkyl and S(=O)₂C₁₋₄-alkyl; aryl or heteroaryl, unsubstituted or mono- or polysubstituted by one or more substituents each independently selected from the group consisting of F, Cl, Br, I, C₁₋₄-alkyl, C(=O)OH, C(=O)OC₁₋₄-alkyl, C(=O)C₁₋₄-alkyl, C(=O)NH-C₁₋₄-alkyl, OH, O-C₁₋₄-alkyl, NH₂, NHC(=O)C₁₋₄-alkyl, NHS(=O)₂C₁₋₄-alkyl, S(=O)₂OH, S(=O)₂OC₁₋₄-alkyl and S(=O)₂C₁₋₄-alkyl; or C₁₋₈-alkyl-bridged aryl or heteroaryl, unsubstituted or mono- or polysubstituted by one or more substituents each independently selected from the group consisting of F, Cl, Br, I, C₁₋₄-alkyl, C(=O)OH, C(=O)OC₁₋₄-alkyl, C(=O)C₁₋₄-alkyl, C(=O)NH-C₁₋₄-alkyl, OH, O-C₁₋₄-alkyl, NH₂, NHC(=O)C₁₋₄-alkyl, NHS (=O)₂C₁₋₄-alkyl, S(=O)₂OH, S(=O)₂OC₁₋₄-alkyl and S(=O)₂C₁₋₄-alkyl; where the alkyl chain may in each case be branched or unbranched, saturated or unsaturated, unsubstituted;
or R⁶ is -C(=O)-CHR^{y}NHR^{z} where
R^{y} is selected from the group consisting of -H, -CH₃, -CH(CH₃)₂, -CH₂CH(CH₃)₂, -CH(CH₃)CH₂CH₃, -CH₂CH₂SCH₃, -CH₂C₆H₅, -CH₂-indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NHC (=NH) NH₂, -CH₂-imidazolyl, -CH₂CO₂H, and -CH₂CH₂CO₂H; and
R^{z} is selected from -H and -C(=O)-C₁₋₈-alkyl.

6. A substituted compound as claimed in any of claims 1-5, **characterized in that**
X¹ and X² are each CH₂;
m and n are each 1;
R¹ is OR⁸ ;
where R⁸ is H; C₁₋₁₀-alkyl, saturated or unsaturated, branched or unbranched, unsubstituted or mono- or polysubstituted by one or more substituents each independently selected from the group consisting of C(=O)OH, C (=O) OC₁₋₄-alkyl, C (=O)C₁₋₄-alkyl, C(=O)NH-C₁₋₄-alkyl, OH, O-C₁₋₄-alkyl, =O, NH₂, NHC(=O)C₁₋₄-alkyl, NHS(=O)₂C₁₋₄-alkyl, S(=O)₂OH, S(=O)₂OC₁₋₄-alkyl and S(=O)₂C₁₋₄-alkyl; or C₁₋₈-alkyl-bridged aryl, unsubstituted or mono- or polysubstituted by one or more substituents each independently selected from the group consisting of F, Cl, Br, I, C₁₋₄-alkyl, C(=O)OH, C(=O)OC₁₋₄-alkyl, C(=O)C₁₋₄-alkyl, C (=O) NH-C₁₋₄-alkyl, OH, O-C₁₋₄-alkyl, NH₂, NHC (=O) C₁₋₄-alkyl, NHS(=O)₂C₁₋₄-alkyl, S(=O)₂OH, S(=O)₂OC₁₋₄-alkyl and S(=O)₂C₁₋₄-alkyl; where the alkyl chain may in each case be branched or unbranched, saturated or unsaturated, unsubstituted;
or R¹ is -NHCHR^{a}C(=O)-R^{b} where
R^{a} is selected from the group consisting of -H, -CH₃, -CH(CH₃)₂, -CH₂CH(CH₃)₂, -CH(CH₃)CH₂CH₃, -CH₂CH₂SCH₃, -CH₂C₆H₅, -CH₂-indolyl, -CH₂OH, -CH(OH)CH₃, -CH₂C(=O)NH₂, CH₂CH₂C(=O) NH₂, -CH₂-C₆H₄-OH, -CH₂SH, -CH₂CH₂CH₂CH₂NH₂, -CH₂CH₂CH₂NHC (=NH) NH₂, -CH₂-imidazolyl, -CH₂CO₂H, and -CH₂CH₂CO₂H; and
R^{b} is selected from -OH and -peptidyl, where the peptidyl is bonded covalently via its N terminus;
R² and R⁵ are each H;
R³ and R⁴ are each H;
R⁶ is H; C₁₋₁₀-alkyl, saturated or unsaturated, branched or unbranched, substituted by one or more substituents each independently selected from the group consisting of C(=O)OH, C(=O)OC₁₋₄-alkyl, C(=O)C₁₋₄-alkyl, C(=O)NH-C₁₋₄-alkyl, OH, O-C₁₋₄-alkyl, =0, NH₂, NHC(=O)C₁₋₄-alkyl and NHS(=O)₂C₁₋₉-alkyl;
or is C(=O)R¹⁰; C(=O)OR¹⁰; C(=O)NR¹⁰R¹¹; S(=O)₂-R¹⁰; S(=O)₂OR¹⁰; or S(=O)₂NR¹⁰R¹¹,
where R¹⁰ and R¹¹ are each independently H; C₁₋₁₀-alkyl, saturated or unsaturated, branched or unbranched, unsubstituted or mono- or polysubstituted by one or more substituents each independently selected from the group consisting of C₁₋₄-alkyl, C(=O)OH, C (=O) OC₁₋₄-alkyl, C (=O) C₁₋₄-alkyl, C(=O)NH-C₁₋₄-alkyl, OH, O-C₁₋₄-alkyl, =O, NH₂, NHC(=O)C₁₋₄-alkyl, NHS (=O)₂C₁₋₄-alkyl, aryl, unsubstituted or mono- or polysubstituted by one or more substituents each independently selected from the group consisting of C₁₄-alkyl, C(=O)OH, C(=O)OC₁₋₄-alkyl, C(=O)C₁₋₄-alkyl, C (=O)NH-C₁₋₄-alkyl, OH, O-C₁₋₄-alkyl, NH₂, NHC(=O)C₁₋₄-alkyl, NHS (=O)₂C₁₋₂-alkyl; or C₁₋₈-alkyl-bridged aryl or heteroaryl, in each case unsubstituted or mono- or polysubstituted by one or more substituents each independently selected from the group consisting of F, Cl, Br, I, C₁₋₄-alkyl, C(=O)OH, C(=O)OC₁₋₄-alkyl, C(=O)C₁₋₄-alkyl, C(=O)NH-C₁₋₄-alkyl, OH, O-C₁₋₄-alkyl, NH₂, NHC (=O)C₁₋₄-alkyl, NHS(=O)₂C₁₋₄-alkyl, where the alkyl chain may in each case be branched or unbranched, saturated or unsaturated, unsubstituted;
or R⁶ is -C(=O)-CHR^{y}NHR^{z} where
R^{y} is selected from the group consisting of -H, -CH₃, -CH(CH₃)₂, -CH₂CH(CH₃)₂, -CH (CH₃)CH₂CH₃, -CH₂CH₂SCH₃, -CH₂C₆H₅, -CH₂-indolyl, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂, -CH₂CH₂CH₂NHC (=NH)NH₂, -CH₂-imidazolyl, -CH₂CO₂H, and -CH₂CH₂CO₂H; and
R^{z} is selected from -H and -C(=O)-C₁₋₈-alkyl.

7. The use of at least one compound of the general formula (I) as claimed in any of claims 1-6 for the induction of an α-helix conformation in at least one protein and/or peptide.

8. A medicament comprising at least one compound of the general formula (I) as claimed in any of claims 1-6
and optionally one or more pharmaceutically acceptable excipients and/or optionally further active ingredients.

9. The use of at least one substituted compound of the general formula (I) as claimed in any of claims 1-6 for production of a medicament for treatment and/or prophylaxis of one or more diseases and/or disorders associated at least partly with the induction of an α-helix conformation in at least one protein and/or at least one peptide.

10. The use of at least one substituted compound of the general formula (I) as claimed in any of claims 1-6 for production of a medicament for treatment and/or prophylaxis of one or more diseases and/or disorders selected from the group consisting of bacterial and viral infection diseases, neurodegenerative disorders and tumor disorders.

11. The use of at least one substituted compound of the general formula (I) as claimed in any of claims 1-6 for production of a substance library of α-helix-induced proteins and/or peptides.

12. A substance library of α-helix-induced proteins and/or peptides comprising at least one substituted compound of the general formula (I) as claimed in any of claims 1-6.

13. A process for producing a peptide library, comprising the step of:
introducing a compound as claimed in any of claims 1-6 into a peptide sequence.

14. The process as claimed in claim 13, **characterized in that** the compound as claimed in any of claims 1-6 is flanked immediately by a glycine residue on each side within the sequence.

15. A peptide library obtainable according to claim 13 or 14.

## Revendications

1. Composés substitués de formule générale (I) 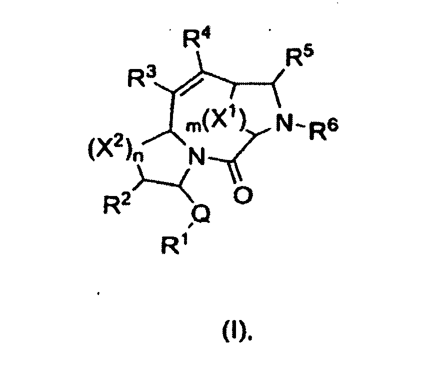 dans laquelle
Q représente C(=O) ou S(=O)₂ ;
X¹ et X² représentent à chaque fois indépendamment l'un de l'autre O, S, CH₂ ou CHR⁷,
R⁷ représentant alkyle en C₁₋₁₀, saturé ou insaturé, ramifié ou non ramifié, non substitué ou substitué une ou plusieurs fois ;
m représente 1, 2 ou 3 ;
n représente 0, 1, 2 ou 3 ;
R¹ représente alkyle en C₁₋₁₀, saturé ou insaturé, ramifié ou non ramifié, non substitué ou substitué une ou plusieurs fois ; cycloalkyle en C₃₋₁₀ ou hétérocyclyle, à chaque fois saturé ou insaturé, non substitué ou substitué une ou plusieurs fois ; aryle ou hétéroaryle, à chaque fois non substitué ou substitué une ou plusieurs fois ; cycloalkyle en C₃₋₁₀ ou hétérocyclyle ponté par alkyle en C₁₋₈, à chaque fois saturé ou insaturé, non substitué ou substitué une ou plusieurs fois, la chaîne alkyle pouvant à chaque fois être ramifiée ou non ramifiée, saturée ou insaturée, non substituée, substituée une ou plusieurs fois ; ou aryle ou hétéroaryle ponté par alkyle en C₁₋₈, à chaque fois non substitué ou substitué une ou plusieurs fois, la chaîne alkyle pouvant à chaque fois être ramifiée ou non ramifiée, saturée ou insaturée, non substituée, substituée une ou plusieurs fois ; OR⁸ ; NR⁸R⁹ ; NHC(=O)R⁸ ; NR⁸R⁹ ou NH-S(=O)₂R⁸ ;
R⁸ et R⁹ représentant à chaque fois indépendamment l'un de l'autre H ; alkyle en C₁₋₁₀, saturé ou insaturé, ramifié ou non ramifié, non substitué ou substitué une ou plusieurs fois ; cycloalkyle en C₃₋₁₀ ou hétérocyclyle, à chaque fois saturé ou insaturé, non substitué ou substitué une ou plusieurs fois ; aryle ou hétéroaryle, à chaque fois non substitué ou substitué une ou plusieurs fois ; cycloalkyle en C₃₋₁₀ ou hétérocyclyle ponté par alkyle en C₁₋₈, à chaque fois saturé ou insaturé, non substitué ou substitué une ou plusieurs fois, la chaîne alkyle pouvant à chaque fois être ramifiée ou non ramifiée, saturée ou insaturée, non substituée, substituée une ou plusieurs fois ; ou aryle ou hétéroaryle ponté par alkyle en C₁₋₈, à chaque fois non substitué ou substitué une ou plusieurs fois, la chaîne alkyle pouvant à chaque fois être ramifiée ou non ramifiée, saturée ou insaturée, non substituée, substituée une ou plusieurs fois ;
ou R¹ représente -NHCHR^{a}C(=O)-R^{b},
R^{a} étant choisi dans le groupe constitué par -H,-CH₃, -CH(CH₃)₂, -CH₂CH(CH₃)₂, -CH(CH₃)CH₂CH₃, -CH₂CH₂SCH₃, -CH₂C₆H₅, -CH₂-indolyle, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂,-CH₂CH₂CH₂NHC (=NH)NH₂, -CH₂-imidazolyle, -CH₂CO₂H et - CH₂CH₂CO₂H ; et
R^{b} étant choisi parmi -OH et -peptidyle, peptidyle étant relié de manière covalente par sa terminaison N ; R² et R⁵ représentent à chaque fois indépendamment l'un de l'autre H ; alkyle en C₁₋₁₀, saturé ou insaturé, ramifié ou non ramifié, non substitué ou substitué une ou plusieurs fois ;
R³ et R⁴ représentent à chaque fois indépendamment l'un de l'autre H ; alkyle en C₁₋₁₀, saturé ou insaturé, ramifié ou non ramifié, non substitué ou substitué une ou plusieurs fois ; aryle ou hétéroaryle, à chaque fois non substitué ou substitué une ou plusieurs fois ;
R⁶ représente H ; alkyle en C₁₋₁₀, saturé ou insaturé, ramifié ou non ramifié, non substitué ou substitué une ou plusieurs fois ; cycloalkyle en C₃₋₁₀ ou hétérocyclyle, à chaque fois saturé ou insaturé, non substitué ou substitué une ou plusieurs fois ; aryle ou hétéroaryle, à chaque fois non substitué ou substitué une ou plusieurs fois ; cycloalkyle en C₃₋₁₀ ou hétérocyclyle ponté par alkyle en C₁₋₈, à chaque fois saturé ou insaturé, non substitué ou substitué une ou plusieurs fois, la chaîne alkyle pouvant à chaque fois être ramifiée ou non ramifiée, saturée ou insaturée, non substituée, substituée une ou plusieurs fois ; ou aryle ou hétéroaryle ponté par alkyle en C₁₋₈, à chaque fois non substitué ou substitué une ou plusieurs fois, la chaîne alkyle pouvant à chaque fois être ramifiée ou non ramifiée, saturée ou insaturée, non substituée, substituée une ou plusieurs fois ; C(=O)R¹⁰ ; C(=O)OR¹⁰ ; C(=O)NR¹⁰R¹¹ ; S(=O)₂-R¹⁰ ; S(=O)₂OR¹⁰ ou S(=O)₂NR¹⁰R¹¹
R¹⁰ et R¹¹ représentant à chaque fois indépendamment l'un de l'autre H ; alkyle en C₁₋₁₀, saturé ou insaturé, ramifié ou non ramifié, non substitué ou substitué une ou plusieurs fois ; cycloalkyle en C₃₋₁₀ ou hétérocyclyle, à chaque fois saturé ou insaturé, non substitué ou substitué une ou plusieurs fois ; aryle ou hétéroaryle, à chaque fois non substitué ou substitué une ou plusieurs fois ; cycloalkyle en C₃₋₁₀ ou hétérocyclyle ponté par alkyle en C₁₋₈, à chaque fois saturé ou insaturé, non substitué ou substitué une ou plusieurs fois, la chaîne alkyle pouvant à chaque fois être ramifiée ou non ramifiée, saturée ou insaturée, non substituée, substituée une ou plusieurs fois ; ou aryle ou hétéroaryle ponté par alkyle en C₁₋₈, à chaque fois non substitué ou substitué une ou plusieurs fois, la chaîne alkyle pouvant à chaque fois être ramifiée ou non ramifiée, saturée ou insaturée, non substituée, substituée une ou plusieurs fois ;
ou R⁶ représente -C (=O) -CHR^{y}NHR^{z},
R^{y} étant choisi dans le groupe constitué par -H,-CH₃, -CH(CH₃)₂, -CH₂CH(CH₃)₂, -CH(CH₃)CH₂CH₃, -CH₂CH₂SCH₃, -CH₂C₆H₅, -CH₂-indolyle, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂,-CH₂CH₂CH₂NHC (=NH)NH₂, -CH₂-imidazolyle, -CH₂CO₂H et - CH₂CH₂CO₂H ; et
R^{z} étant choisi parmi -H et -C(=O)-alkyle en C₁₋₈ ; « alkyle substitué », « hétérocyclyle substitué » et « cycloalkyle substitué » représentant sur les radicaux correspondants la substitution d'un ou de plusieurs atomes d'hydrogène à chaque fois indépendamment les uns des autres par F ; Cl ; Br ; I ; NO₂ ; CN ; CF₃ ; alkyle en C₁₋₈ ; phényle ; C(=O)OH ; C(=O)O-alkyle en C₁₋₈ ; C(=O)O-phényle ; C(=O)NH₂ ; C(=O)NH-alkyle en C₁₋₈ ; C(=O)NH-phényle; C(=O)N(alkyle en C₁₋₈)₂ ; OH ; =O ; OCF₃ ; O-alkyle en C₁₋₈ ; O-C(=O)-alkyle en C₁₋₈ ; SH ; SCF₃ ; S-alkyle en C₁₋₈ ; S(=O)₂OH ; S(=O)₂-alkyle en C₁₈ ; S(=O)₂O-alkyle en C₁₋₈ ; S(=O)₂NH-alkyle en C₁₋₈ ; S(=O)₂N(alkyle en C₁₋₈)₂ ; NH₂ ; NH-alkyle en C₁₋₈ ; NH-phényle ; N (alkyle en C₁₋₈)₂ ; NH-C(=O)-alkyle en C₁₋₈ ; NH-S(=O)₂-alkyle en C₁₋₈ ;
« aryle substitué » et « hétéroaryle substitué » représentant sur les radicaux correspondants la substitution d'un ou de plusieurs atomes d'hydrogène à chaque fois indépendamment les uns des autres par F ; Cl ; Br ; I ; NO₂ ; CN ; CF₃ ; alkyle en C₁₋₈ ; phényle ; C(=O)OH ; C(=O)O-alkyle en C₁₋₈ ; C(=O)O-phényle ; C(=O)NH₂ ; C(=O)NH-alkyle en C₁₋₈ ; C(=O)NH-phényle ; C(=O)N(alkyle en C₁₋₈)₂ ; OH ; OCF₃ ; O-alkyle en C₁₋₈ ; O-C(=O)-alkyle en C₁₋₈ ; SH ; SCF₃ ; S-alkyle en C₁₋₈ ; S(=O)₂OH ; S(=O)₂-alkyle en C₁₋₈ ; S(=O)₂O-alkyle en C₁₈ ; S(=O)₂NH-alkyle en C₁₋₈ ; S(=O)₂N(alkyle en C₁₋₈)₂ ; NH₂ ; NH-alkyle en C₁₋₈ ; NH-phényle ; N (alkyle en C₁₈)₂ ; NH-C(=O)-alkyle en C₁₋₈ ; NH-S(=O)₂-alkyle en C₁₋₈ ; à chaque fois sous la forme des composés libres ; des racémats, des énantiomères, diastéréomères, mélanges des énantiomères ou diastéréomères ou d'un énantiomère ou diastéréomère individuel ; ou sous la forme des sels d'acides ou de bases physiologiquement acceptables ; ou éventuellement sous la forme de solvates.

2. Composés substitués selon la revendication 1, **caractérisés en ce que**
X¹ et X² représentent à chaque fois CH₂ ;
m représente 1 ou 2, de préférence 1 ;
n représente 0, 1 ou 2, de préférence 1.

3. Composés substitués selon la revendication 1 ou 2, **caractérisés en ce que**
R¹ représente OR⁸ ; NHR⁸ ou NHC(=O)R⁸ ;
R⁸ représentant H ; alkyle en C₁₋₁₀, saturé ou insaturé, ramifié ou non ramifié, non substitué ou substitué une ou plusieurs fois avec un ou plusieurs substituants choisis à chaque fois indépendamment les uns des autres dans le groupe constitué par F, Cl, Br, I, C(=O)OH, C(=O)O-alkyle en C₁₋₄, C(=O)-alkyle en C₁₋₄, C(=O)NH-alkyle en C₁₋₄, OH, O-alkyle en C₁₋₄, =0, NH₂, NHC(=O)-alkyle en C₁₋₄, NHS(=O)₂-alkyle en C₁₋₄, S(=O)₂OH, S(=O)₂O-alkyle en C₁₋₄ et S(=O)₂-alkyle en C₁₋₄ ; aryle, non substitué ou substitué une ou plusieurs fois avec un ou plusieurs substituants choisis à chaque fois indépendamment les uns des autres dans le groupe constitué par F, Cl, Br, I, alkyle en C₁₋₄, C(=O)OH, C(=O)O-alkyle en C₁₋₄, C(=O)-alkyle en C₁₋₄, C(=O)NH-alkyle en C₁₋₄, OH, O-alkyle en C₁₋₄, NH₂, NHC(=O)-alkyle en C₁₋₄, NHS(=O)₂-alkyle en C₁₋₄, S(=O)₂OH, S(=O)₂O-alkyle en C₁₋₄ et S(=O)₂-alkyle en C₁₋₄ ; ou aryle ponté par alkyle en C₁₋₈, non substitué ou substitué une ou plusieurs fois avec un ou plusieurs substituants choisis à chaque fois indépendamment les uns des autres dans le groupe constitué par F, Cl, Br, I, alkyle en C₁₄, C(=O)OH, C(=O)O-alkyle en C₁₋₄, C(=O)-alkyle en C₁₋₄, C(=O)NH-alkyle en C₁₋₄, OH, O-alkyle en C₁₋₄, NH₂, NHC(=O)-alkyle en C₁₋₄, NHS(=O)₂-alkyle en C₁₋₄, S(=O)₂OH, S (=O)₂O-alkyle en C₁₋₄ et S (=O)₂-alkyle en C₁₋₄ ; la chaîne alkyle pouvant à chaque fois être ramifiée ou non ramifiée, saturée ou insaturée, non substituée ;
ou R¹ représente -NHCHR^{a}C(=O)-R^{b},
R^{a} étant choisi dans le groupe constitué par -H,-CH₃, -CH(CH₃)₂, -CH₂CH(CH₃)₂, -CH(CH₃)CH₂CH₃, -CH₂CH₂SCH₃, -CH₂C₆H₅, -CH₂-indolyle, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂,-CH₂CH₂CH₂NHC (=NH) NH₂, -CH₂-imidazolyle, -CH₂CO₂H et - CH₂CH₂CO₂H ; et
R^{b} étant choisi parmi -OH et -peptidyle, peptidyle étant relié de manière covalente par sa terminaison N.

4. Composés substitués selon l'une quelconque des revendications 1 à 3, **caractérisés en ce que**
R² et R⁵ représentent à chaque fois indépendamment l'un de l'autre H ; alkyle en C₁-C₁₀, saturé ou insaturé, ramifié ou non ramifié ; non substitué ;
R³ et R⁴ représentent à chaque fois indépendamment l'un de l'autre H ; alkyle en C₁-C₁₀, saturé ou insaturé, ramifié ou non ramifié ; non substitué.

5. Composés substitués selon l'une quelconque des revendications 1 à 4, **caractérisés en ce que** R⁶ représente H ; alkyle en C₁₋₁₀, saturé ou insaturé, ramifié ou non ramifié, substitué avec un ou plusieurs substituants choisis à chaque fois indépendamment les uns des autres dans le groupe constitué par F, Cl, Br, I, C(=O)OH, C(=O)O-alkyle en C₁₋₄, C(=O)-alkyle en C₁₋₄, C(=O)NH-alkyle en C₁₋₄, OH, O-alkyle en C₁₋₄, =0, NH₂, NHC(=O)-alkyle en C₁₋₄, NHS(=O)₂-alkyle en C₁₋₄, S(=O)₂OH, S(=O)₂O-alkyle en C₁₋₄ et S(=O)₂-alkyle en C₁₋₄ ;
ou C(=O)R¹⁰ ; C(=O)OR¹⁰ ; C(=O)NR¹⁰R¹¹ ; S(=O)₂-R¹⁰; S (=O)₂OR¹⁰ ou S (=O)₂NR¹⁰R¹¹ ;
R¹⁰ et R¹¹ représentant à chaque fois indépendamment l'un de l'autre H ; alkyle en C₁₋₁₀, saturé ou insaturé, ramifié ou non ramifié, non substitué ou substitué une ou plusieurs fois avec un ou plusieurs substituants choisis à chaque fois indépendamment les uns des autres dans le groupe constitué par F, Cl, Br, I, alkyle en C₁₄, C(=O)OH, C(=O)O-alkyle en C₁₋₄, C(=O)-alkyle en C₁₋₄, C(=O)NH-alkyle en C₁₋₄, OH, O-alkyle en C₁₋₄, =O, NH₂, NHC(=O)-alkyle en C₁₋₄, NHS(=O)₂-alkyle en C₁₋₄, S(=O)₂OH, S(=O)₂O-alkyle en C₁₋₄ et S(=O)₂-alkyle en C₁₋₄ ; aryle ou hétéroaryle, à chaque fois non substitué ou substitué une ou plusieurs fois avec un ou plusieurs substituants choisis à chaque fois indépendamment les uns des autres dans le groupe constitué par F, Cl, Br, I, alkyle en C₁₋₄, C(=O)OH, C(=O)O-alkyle en C₁₋₄, C(=O)-alkyle en C₁₋₄, C(=O)NH-alkyle en C₁₋₄, OH, O-alkyle en C₁₋₄, NH₂, NHC(=O)-alkyle en C₁₋₄, NHS(=O)₂-alkyle en C₁₋₄, S(=O)₂OH, S(=O)₂O-alkyle en C₁₋₄ et S(=O)₂-alkyle en C₁₋₄ ; ou aryle ou hétéroaryle ponté par alkyle en C₁₋₈, à chaque fois non substitué ou substitué une ou plusieurs fois avec un ou plusieurs substituants choisis à chaque fois indépendamment les uns des autres dans le groupe constitué par F, Cl, Br, I, alkyle en C₁₋₄, C(=O)OH, C(=O)O-alkyle en C₁₋₄, C(=O)-alkyle en C₁₋₄, C(=O)NH-alkyle en C₁₋₄, OH, O-alkyle en C₁₋₄, NH₂, NHC(=O)-alkyle en C₁₋₄, NHS(=O)₂-alkyle en C₁₋₄, S(=O)₂OH, S(=O)₂O-alkyle en C₁₋₄ et S(=O)₂-alkyle en C₁₋₄ ; la chaîne alkyle pouvant à chaque fois être ramifiée ou non ramifiée, saturée ou insaturée, non substituée ;
ou R⁶ représente -C (=O)-CHR^{y}NHR^{z},
R^{y} étant choisi dans le groupe constitué par -H,-CH₃, -CH(CH₃)₂, -CH₂CH(CH₃)₂, -CH(CH₃)CH₂CH₃, -CH₂CH₂SCH₃,-CH₂C₆H₅, -CH₂-indolyle, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂,-CH₂CH₂CH₂NHC (=NH) NH₂, -CH₂-imidazolyle, -CH₂CO₂H et - CH₂CH₂CO₂H ; et
R^{z} étant choisi parmi -H et -C(=O)-alkyle en C₁₋₈.

6. Composés substitués selon l'une quelconque des revendications 1 à 5, **caractérisés en ce que**
X¹ et X² représentent à chaque fois CH₂ ;
m et n représentent à chaque fois 1 ;
R¹ représente OR⁸,
R⁸ représentant H ; alkyle en C₁₋₁₀ saturé ou insaturé, ramifié ou non ramifié, non substitué ou substitué une ou plusieurs fois avec un ou plusieurs substituants choisis à chaque fois indépendamment les uns des autres dans le groupe constitué par C(=O)OH, C(=O)O-alkyle en C₁₋₄, C(=O)-alkyle en C₁₋₄, C(=O)NH-alkyle en C₁₋₄, OH, O-alkyle en C₁₋₄, =0, NH₂, NHC(=O)-alkyle en C₁₋₄, NHS(=O)₂-alkyle en C₁₋₄, S(=O)₂OH, S(=O)₂O-alkyle en C₁₋₄ et S(=O)₂-alkyle en C₁₋₄ ; ou aryle ponté par alkyle en C₁₋₈, non substitué ou substitué une ou plusieurs fois avec un ou plusieurs substituants choisis à chaque fois indépendamment les uns des autres dans le groupe constitué par F, Cl, Br, I, alkyle en C₁₄, C(=O)OH, C(=O)O-alkyle en C₁₋₄, C(=O)-alkyle en C₁₋₄, C(=O)NH-alkyle en C₁₋₄, OH, O-alkyle en C₁₋₄, NH₂, NHC(=O)-alkyle en C₁₋₄, NHS(=O)₂-alkyle en C₁₋₄, S(=O)₂OH, S(=O)₂O-alkyle en C₁₋₄ et S (=O)₂-alkyle en C₁₋₄ ; la chaîne alkyle pouvant à chaque fois être ramifiée ou non ramifiée, saturée ou insaturée, non substituée ;
ou R¹ représente -NHCHR^{a}C(=O)-R^{b},
R^{a} étant choisi dans le groupe constitué par -H, - CH₃, -CH(CH₃)₂, -CH₂CH (CH₃)₂, -CH (CH₃)CH₂CH₃, -CH₂CH₂SCH₃,-CH₂C₆H₅, -CH₂-indolyle, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂,-CH₂CH₂CH₂NHC (=NH) NH₂, -CH₂-imidazolyle, -CH₂CO₂H et - CH₂CH₂CO₂H ; et
R^{b} étant choisi parmi -OH et -peptidyle, peptidyle étant relié de manière covalente par sa terminaison N ;
R² et R⁵ représentent à chaque fois H ;
R³ et R⁴ représentent à chaque fois H ;
R⁶ représente H ; alkyle en C₁₋₁₀, saturé ou insaturé, ramifié ou non ramifié, substitué avec un ou plusieurs substituants choisis à chaque fois indépendamment les uns des autres dans le groupe constitué par C(=O)OH, C(=O)O-alkyle en C₁₋₄, C(=O)-alkyle en C₁₋₄, C(=O)NH-alkyle en C₁₋₄, OH, O-alkyle en C₁₋₄, =0, NH₂, NHC(=O)-alkyle en C₁₋₄ et NHS(=O)₂-alkyle en C₁₋₄ ;
ou C(=O)R¹⁰ ; C(=O)OR¹⁰ ; C(=O)NR¹⁰R¹¹ ; S(=O)₂-R¹⁰ ;
S(=O)₂OR¹⁰ ou S (=O)₂NR¹⁰R¹¹,
R¹⁰ et R¹¹ représentant à chaque fois indépendamment l'un de l'autre H ; alkyle en C₁₋₁₀, saturé ou insaturé, ramifié ou non ramifié, non substitué ou substitué une ou plusieurs fois avec un ou plusieurs substituants choisis à chaque fois indépendamment les uns des autres dans le groupe constitué par alkyle en C₁₋₄, C(=O)OH, C(=O)O-alkyle en C₁₋₄, C(=O)-alkyle en C₁₋₄, C(=O)NH-alkyle en C₁₋₄, OH, O-alkyle en C₁₋₄, =0, NH₂, NHC(=O)-alkyle en C₁₋₄, NHS(=O)₂-alkyle en C₁₋₄ ; aryle, non substitué ou substitué une ou plusieurs fois avec un ou plusieurs substituants choisis à chaque fois indépendamment les uns des autres dans le groupe constitué par alkyle en C₁₋₄, C(=O)OH, C(=O)O-alkyle en C₁₋₄, C(=O)-alkyle en C₁₋₄, C(=O)NH-alkyle en C₁₋₄, OH, 0-alkyle en C₁₋₄, NH₂, NHC(=O)-alkyleen C₁₋₄, NHS(=O)₂-alkyle en C₁₋₄ ; ou aryle ou hétéroaryle ponté par alkyle en C₁₋₈, à chaque fois non substitué ou substitué une ou plusieurs fois avec un ou plusieurs substituants choisis à chaque fois indépendamment les uns des autres dans le groupe constitué par F, Cl, Br, I, alkyle en C₁₄, C(=O)OH, C(=O)O-alkyle en C₁₋₄, C(=O)-alkyle en C₁₋₄, C(=O)NH-alkyle en C₁₋₄, OH, O-alkyle en C₁₋₄, NH₂, NHC(=O)-alkyle en C₁₋₄, NHS(=O)₂-alkyle en C₁₋₄ ; la chaîne alkyle pouvant à chaque fois être ramifiée ou non ramifiée, saturée ou insaturée, non substituée ; ou R⁶ représente -C(=O)-CHR^{y}NHR^{z},
R^{y} étant choisi dans le groupe constitué par -H,-CH₃, -CH(CH₃)₂, -CH₂CH(CH₃)₂, -CH(CH₃)CH₂CH₃, -CH₂CH₂SCH₃, -CH₂C₆H₅, -CH₂-indolyle, -CH₂OH, -CH(OH)CH₃, -CH₂C(=O)NH₂, CH₂CH₂C(=O)NH₂, -CH₂-C₆H₄-OH, -CH₂SH, -CH₂CH₂CH₂CH₂NH₂,-CH₂CH₂CH₂NHC (=NH) NH₂, -CH₂-imidazolyle, -CH₂CO₂H et-CH₂CH₂CO₂H ; et
R^{z} étant choisi parmi -H et -C(=O)-alkyle en C₁₋₈.

7. Utilisation d'au moins un composé de formule générale (I) selon l'une quelconque des revendications 1 à 6 pour l'induction d'une conformation d'hélice α dans au moins une protéine et/ou un peptide.

8. Médicament contenant au moins un composé de formule générale (I) selon l'une quelconque des revendications 1 à 6, ainsi qu'éventuellement un ou plusieurs adjuvants pharmaceutiquement compatibles et/ou éventuellement d'autres agents actifs.

9. Utilisation d'au moins un composé substitué de formule générale (I) selon l'une quelconque des revendications 1 à 6 pour la fabrication d'un médicament pour le traitement et/ou la prophylaxie d'une ou de plusieurs maladies et/ou d'un ou de plusieurs troubles, associés au moins en partie avec l'induction d'une conformation d'hélice α dans au moins une protéine et/ou dans au moins un peptide.

10. Utilisation d'au moins un composé substitué de formule générale (I) selon l'une quelconque des revendications 1 à 6 pour la fabrication d'un médicament pour le traitement et/ou la prophylaxie d'une ou de plusieurs maladies et/ou d'un ou de plusieurs troubles, choisis dans le groupe constitué par les maladies infectieuses bactériennes et virales, les maladies neurodégénératives et les maladies tumorales.

11. Utilisation d'au moins un composé substitué de formule générale (I) selon l'une quelconque des revendications 1 à 6 pour la fabrication d'une bibliothèque de substances de protéines et/ou de peptides à hélice α induite.

12. Bibliothèque de substances de protéines et/ou de peptides à hélice α induite contenant au moins un composé substitué de formule générale (I) selon l'une quelconque des revendications 1 à 6.

13. Procédé de fabrication d'une bibliothèque de peptides comprenant l'étape suivante :
l'introduction d'un composé selon l'une quelconque des revendications 1 à 6 dans une séquence peptidique.

14. Procédé selon la revendication 13, **caractérisé en ce que** le composé selon l'une quelconque des revendications 1 à 6 est flanqué à chaque fois directement par un radical glycine des deux côtés dans la séquence.

15. Bibliothèque de peptides pouvant être obtenue selon la revendication 13 ou 14.
